# EUROPÄISCHE PATENTSCHRIFT

(11) **EP 3 938 014 B1**
(45) Veröffentlichungstag und Bekanntmachung des Hinweises auf die Patenterteilung: **04.06.2025**
(21) Anmeldenummer: 20706749.7
(22) Anmeldetag: 02.03.2020
(51) Int. Cl.: A61M 5/315

(54) **DOSIEREINRICHTUNG FÜR EINE INJEKTIONSVORRICHTUNG**
METERING DEVICE FOR AN INJECTION DEVICE
DISPOSITIF DE DOSAGE POUR UN DISPOSITIF D'INJECTION

(30) Priorität: 15.03.2019 EP 19163197
(43) Veröffentlichungstag der Anmeldung: 19.01.2022
(73) Patentinhaber: Ypsomed AG, 3401 Burgdorf (CH)
(72) Erfinder: SCHEURER, Simon, 3006 Bern (CH); HOSTETTLER, Patrick, 3415 Hasle (CH); HIRSCHEL, Jürg, 3007 Bern (CH)
(74) Vertreter: Meier Obertüfer, Jürg
(86) Internationale Anmeldenummer: PCT/EP2020/055385
(87) Internationale Veröffentlichungsnummer: WO 2020/187547

(56) Entgegenhaltungen:
- US-A1- 2015 088 079
- US-A1- 2015 112 274
- US-A1- 2017 319 789

## Beschreibung

### TECHNISCHES GEBIET

Die vorliegende Erfindung betrifft das Gebiet der medizinischen Injektionsvorrichtungen zur Verabreichung von flüssigen Substanzen insbesondere von Medikamenten oder medizinischen Substanzen wie Insulin- und Hormonpräparationen. Die Erfindung bezieht sich auf eine Dosiereinrichtung für eine Injektionsvorrichtung mit einem Dosiseinstellelement zum Einstellen einer Dosis und mit einer Kupplungshülse zum wahlweisen koppeln des Dosiseinstellelements mit einer Antriebseinrichtung zum Ausschütten der Dosis.

### HINTERGRUND DER ERFINDUNG

Bekannte Injektionsgeräte umfassen üblicherweise eine Antriebshülse zum Antreiben einer Kolbenstange um die flüssigen Substanz aus einer Karpule oder einem Produktbehälter auszuschütten, eine Dosierhülse zum Einstellen einer zu verabreichenden Dosis und eine Kupplungshülse zum wahlweisen direkten oder indirekten koppeln der Dosierhülse mit der Antriebshülse.

Um eine zu verabreichende Dosis einzustellen, dreht oder zeiht der Benutzer an der Dosierhülse, welche sich dabei aus dem Gehäuse des Injektionsgeräts herausschraubt bzw. herausbewegt. Damit die eingestellte Dosis ausgeschüttet werden kann, drückt der Benutzer an einem proximalen Ende des Injektionsgeräts auf einen Ausschüttknopf oder erzeugt eine Kraft in distaler Richtung, wodurch die Dosierhülse in das Gehäuse eingeschraubt oder eingeschoben wird. Im Unterschied zum Einstellen einer Dosis überträgt beim Ausschütten die Kupplungshülse die Dreh- oder Schiebebewegung der Dosierhülse auf die Antriebshülse, welche dadurch die Kolbenstange antreibt und die Substanz ausschüttet. Je nach Ausführung ist dabei Kolbenstange drehbar oder nur verschiebbar relativ zum Gehäuse.

Um eine versehentlich zu hoch eingestellte Dosis zu korrigieren, kann der Benutzer die Dosierhülse in das Gehäuse zurückdrehen oder zurückschieben. Bei dieser Korrekturbewegung wie auch bei der Handhabung des Injektionsgeräts zwischen den Verabreichungen muss sichergestellt werden, dass sich die Position der Kolbenstange nicht ungewollt relativ zur Karpule verstellen kann, insbesondere dass sich die Kolbenstange nicht von der Karpule wegbewegt.

Eine Möglichkeit ein ungewolltes Verstellen zu verhindern, ist die Verwendung einer sogenannten Rückdrehsicherung. Diese verhindert entweder direkt das Rotieren der Kolbenstange entgegen der Rotationsrichtung zum Ausschütten der Substanz oder die Rückdrehsicherung greift am antreibenden Antriebselement an, so dass eine Rückbewegung der Kolbenstange indirekt verhindert wird. Letzteres hat den Vorteil, dass die Rückdrehsicherung auch bei Kolbenstangen zur Anwendung gelangen kann, welche bei der Verabreichung eine ausschliesslich schiebende Bewegung absolvieren.

Ein stiftförmiges Injektionsgerät mit einer Dosiereinrichtung mit einer oben erwähnten Anordnung mit Dosier- und Kupplungshülse beschreibt beispielsweise die EP 1 003 581 B1. Das Gehäuse des Injektionsgeräts weist ein Innengewinde auf, in welches die Kolbenstange eingeschraubt ist. Nach dem Einstellen der Dosis wird die Dosierhülse mittels eines Knopfs in das Gehäuse gedrückt, wodurch die in der Dosierhülse befindliche Kupplungshülse mit der Dosierhülse gekoppelt wird, so dass die Drehbewegung auf die koaxial innerhalb der Kupplungshülse angeordnete Antriebshülse übertragen wird. Die Antriebshülse ist axial fest im Gehäuse gelagert. Sie schraubt durch ihre Drehbewegung die Kolbenstange durch das Innengewinde. Dadurch wird ein Flansch am Ende der Kolbenstange auf einen Stopfen in einer Karpule gedrückt, wodurch das Produkt aus der Karpule ausgeschüttet wird. Die Antriebshülse weist radial ausragende Arme auf, welche mit gehäusefesten Sägezähnen zusammenwirken und dadurch eine Ratsche bilden. Aufgrund der Sägezahnform können die Arme nur in einer Richtung über die Zähne bewegt werden und sperren in der entgegengesetzten Richtung. Somit ist eine Rückdrehsicherung realisiert, bei der die Antriebshülse und damit die Kolbenstange nur in Richtung zum Ausschütten drehbar sind.

Bei dieser Konstruktion der Rückdrehsicherung müssen die Arme der Antriebshülse mit einer Vorspannkraft auf die Sägezähne gedrückt werden, damit die Rückdrehsicherung funktioniert und die Antriebshülse nicht dennoch in Gegenrichtung gedreht werden kann. Die Vorspannung hat zur Folge, dass durch die Reibung bei der Drehbewegung beim Ausschütten ein erhöhter Kraftaufwand bzw. ein erhöhtes Drehmoment aufgebracht werden muss.

Eine weitere Möglichkeit einer Rückdrehsicherung offenbart die EP 2 262 533 B1. Bei der beschriebenen Injektionsvorrichtung handelt es sich um einen sogenannten Autopen, bei dem die Kolbenstange zum Ausschütten nicht durch eine vom Benutzer aufgebrachte Kraft, sondern durch eine von einer vorgespannte Feder erzeugten Federkraft angetrieben wird. Die Antriebshülse, welche die Kolbenstange antreibt, weist einen Nocken auf, welcher in eine Arretierhülse eingreift. Die Arretierhülse hält dadurch die Antriebshülse rotationsfest relativ zum Gehäuse. Dabei ist die Arretierhülse rotationsfest aber axial verschiebbar relativ zum Gehäuse gelagert. Zum Ausschütten der Dosis wird die Arretierhülse in axialer Richtung relativ zur Antriebshülse über den Nocken geschoben und gibt dadurch die Antriebshülse rotativ frei, so dass die Antriebshülse angetrieben durch die vorgespannte Feder rotieren kann und die Kolbenstange zum Ausschütten antreiben kann.

Diese Konstruktion ist auf federangetriebene Injektionsgeräte beschränkt, bei denen keine Dosierhülse in axialer Richtung relativ zum Gehäuse verschoben werden muss zum Einstellen einer Dosis. Für Injektionsgeräte mit einer Dosierhülse, wie sie beispielsweise in der oben genannten EP 1 003 581 B1 beschrieben sind, funktioniert eine solche Rückdrehsicherung mit einer Arretierhülse nicht, da die Dosierhülse relativ zum Gehäuse verschoben wird beim Einstellen resp. Ausschütten einer Dosis.

Die US 2015/0112274 offenbart einen Injektionspen, welcher einen Dosiseinstellknopf, eine Antriebshülse und eine Ratschenscheibe. Die Antriebshülse weist am distalen Ende einen Flansch auf, welcher Sägezähne umfasst. Eine Feder spannt die Ratschenscheibe gegen den Flansch vor. Beim Aufdosieren wirken Stoppflächen der Sägezähne der Antriebshülse mit Stoppflächen von Sägezähne der Ratschenscheibe zusammen und verhindern so, dass die Antriebshülse rotiert. Eine Drehung des Dosiseinstellknopfs in Dosierrichtung wird wegen dieser aus den Sägezähnen gebildeten Einwegratsche zwischen Antriebshülse und Ratschenscheibe nicht auf das Rücksetzelement übertragen.

Die US 2017/0319789 offenbart einen Injektionspen mit einer Dosiseinstellhülse und einer Antriebshülse, welche rotationsfest zu einer Kolbenstange gelagert ist. Eine Einwegratsche, welche an einem Gehäusesteg angeordnet ist, verhindert, dass die Kolbenstange während dem Dosiseinstellen rotiert. Zum Ausschütten der eingestellten Dosis wird ein Ausschüttknopf gedrückt, wodurch die Dosiseinstellhülse zusammen mit der Antriebshülse und der Kolbenstange rotieren. US 2015/0088079 beschreibt einen Ausschüttmechanismus für einen wiederverwendbaren Autopen. Dieser umfasst eine Antriebshülse, welche in Gewindeverbindung mit einer Kolbenstange steht. Durch Drucken eines Ausschüttknopfs wird eine Dosierhülse mit der Antriebshülse gekoppelt, so dass beide rotieren. Dadurch wird die Kolbenstange in Ausschüttrichtung geschraubt zum Ausschütten einer zuvor eingestellten Dosis.

### DARSTELLUNG DER ERFINDUNG

Es ist eine Aufgabe der Erfindung bei Injektionsvorrichtungen, bei denen sich beim Einstellen einer Dosis ein Dosiseinstellelement relativ zu einem Gehäuse der Injektionsvorrichtung verschiebt, den Kraftaufwand beim Ausschütten zu minimieren.

Diese Aufgabe wird gelöst durch eine Dosiereinrichtung und einer Injektionsvorrichtung gemäss den unabhängigen Ansprüchen. Bevorzugte Ausführungsformen sind Gegenstand der abhängigen Ansprüche.

Gemäss der Erfindung umfasst die Dosiereinrichtung ein Gehäuse mit einer Längsachse, ein Dosiseinstellelement zum Einstellen der Dosis, ein Halteelement und eine Kupplungshülse zum Antreiben einer Antriebseinrichtung zum Ausschütten der Dosis. Dabei befinden sich das Dosiseinstellelement, das Halteelement und die Kupplungshülse im Gehäuse. Zum Einstellen und Korrigieren der Dosis sind das Dosiseinstellelement und die Kupplungshülse relativ zum Gehäuse verschiebbar und die Kupplungshülse kann mittels des Halteelements rotationsfest relativ zum Gehäuse gehalten werden. Zum Ausschütten der Dosis kann die Kupplungshülse vom Halteelement freigegeben werden, so dass die Kupplungshülse relativ zum Gehäuse rotieren kann, wobei die Dosiereinrichtung im Gehäuse eine Führung umfasst, in welcher das Halteelement insbesondere beim Einstellen und Korrigieren einer Dosis in Richtung der Längsachse relativ zum Gehäuse verschiebbar ist und rotationsfest relativ zum Gehäuse geführt ist. Dabei bedeutet rotationsfest, dass keine Relativdrehung möglich ist. Die Führung ist im Gehäuse selber oder in einem Element innerhalb des Gehäuses ausgebildet.

Da das Halteelement in der Führung axial (d. h. in Richtung der Längsachse des Gehäuses) relativ zum Gehäuse verschiebbar ist, kann sich das Halteelement zusammen mit der Kupplungshülse beim Einstellen oder Korrigieren einer Dosis axial verschieben. Jedoch wird an jeder axialen Position die Kupplungshülse mittels des Halteelements rotationsfest relativ zum Gehäuse gehalten. Dadurch wird ein allfälliges Drehmoment direkt auf das Gehäuse übertragen bzw. an diesem abgestützt. Das bedeutet, die Kupplungshülse kann sich beim Einstellen und Korrigieren nicht relativ zum Gehäuse drehen, wodurch die Antriebseinrichtung nicht angetrieben wird. Ein ungewolltes Ausschütten des Produkts wird dadurch effizient vermieden. Das Halteelement hält wahlweise die Kupplungshülse relativ zum Gehäuse fest oder gibt die Kupplungshülse rotativ vom Gehäuse frei, damit die Kupplungshülse die Antriebseinrichtung antreiben kann und somit die Substanz aus der Karpule oder dem Produktbehälter ausschütten kann.

Beim Ausschütten ist die Kupplungshülse vom Halteelement freigegeben, so dass die Kupplungshülse relativ zum Gehäuse rotierbar ist. Das Halteelement wirkt beim Ausschütten nicht mit der Kupplungshülse zusammen und beeinflusst diese bei ihrer Rotation nicht. Eine aus dem Stand der Technik bekannte Rückdrehsicherung an einer Antriebshülse, welche eine erhöhte Reibung beim der Ausschüttdrehung verursacht, entfällt dadurch. Die erfindungsgemässe Dosiereinrichtung ermöglicht somit eine Rückdrehsicherung, die die Reibung beim Ausschütten nicht zusätzlich erhöht und somit den für das Ausschütten aufzubringenden Kraftaufwand nicht beeinflusst.

Konkret muss eine allfällige Ratsche mit radialen, flexiblen Armen an der Antriebshülse, wie beispielsweise in der EP 2 262 533 B1 offenbart, nicht als Rückdrehsicherung eingesetzt werden. Eine solche Ratsch kann daher ausschliesslich dazu dienen, ein akustisches oder taktiles Signal als Rückmeldung für den Benutzer zu erzeugen. Dadurch können beispielsweise die flexiblen Arme der Ratsche mit weniger Vorspannung auf die Zähne der Ratsche gedrückt werden, wodurch der Kraftaufwand beim Ausschütten, um die Reibung zu überwinden, erheblich reduziert wird.

Die Erfindung umfasst ferner eine Injektionsvorrichtung umfassend einen Karpulenhalter zum Halten einer Karpule mit einer medizinischen Substanz und der Dosiereinrichtung. Vorzugsweise ist die Injektionsvorrichtung ein Injektionspen, insbesondere ein Einweg-Injektionspen. Die Injektionsvorrichtung umfasst in einer bevorzugten Ausführung einen Karpulenhalter zum Halten einer Karpule mit der medizinischen Substanz, eine Nadel oder Kanüle, die am Karpulenhalter anbringbar ist, so dass die medizinische Substanz durch die Nadel oder Kanüle verabreicht werden kann, die Dosiereinrichtung und die Antriebseinrichtung mit einer Kolbenstange, welche weiter unten im Detail beschrieben ist. Bei der Injektionsvorrichtung ist die zu verabreichende Dosis mit dem Dosiseinstellelement einstellbar ist. Die Dosis kann in einer oder mehreren Injektionen erfolgen bis das gesamte in der Injektionsvorrichtung vorhandene Produkt ausgeschüttet ist. Die Injektionsvorrichtung ist vorzugsweise stiftförmig ausgebildet mit einem länglichen Gehäuse, insbesondere mit einem zylindrischen Gehäuse, welches im Querschnitt aber nicht zwingend eine kreisrunde Form aufweisen muss, sondern auch oval oder mehreckig geformt sein kann. Im Gehäuse befindet sich vorzugsweise koaxial angeordnet die Kupplungshülse. Diese hat in einer bevorzugten Ausführung eine längliche, zylindrische Form. Zwischen einer Innenwand des Gehäuses und einer Aussenseite der Kupplungshülse befindet sich vorzugsweise das Halteelement. Dieses ist bevorzugt ebenfalls koaxial im Gehäuse angeordnete und kann beispielsweise hülsen- oder ringförmig ausgeführt sein. Genauso gut kann das Halteelement beispielsweise in der Form einer Scheibe, einer Einlage oder in Form eines in radialer Richtung ausgerichteten Arms ausgeführt sein, welcher die Kupplungshülse relativ zum Gehäuse rotationsfest halten kann.

Das Dosiseinstellelement, das Halteelement und die Kupplungshülse, befinden sich innerhalb des Gehäuses der Dosiereinrichtung. Das schliesst jedoch nicht aus, dass einzelne Element oder Bereiche des Dosiseinstellelements, der Kupplungshülse und des Halteelements aus dem Gehäuse herausragen. Insbesondere befindet sich vorzugsweise ein proximaler Endbereich des Dosiseinstellelements und der Kupplungshülse ausserhalb des Gehäuses.

Die Injektionsvorrichtung umfasst bevorzugt ein Produktbehälter, insbesondere eine Karpule, in dem sich eine medizinische Substanz befindet. Um das Produkt aus dem Produktbehälter auszuschütten, kann beispielsweise mittels einer Kolbenstange der Injektionsvorrichtung ein Stopfen im Produktbehälter in einer Ausschüttrichtung in distaler Richtung verschoben. Vorzugsweise steht diese Ausschüttrichtung parallel zur Richtung der Längsachse des Gehäuses.

In einer Variante der erfindungsgemässen Ausführung kann das Gehäuse weiter einen Gehäuseeinsatz umfassen, welcher vorzugsweise hülsenförmig ausgebildet ist und koaxial zur zentralen Längsachse des Gehäuses im Gehäuse angeordnet ist. Der Gehäuseeinsatz kann beispielsweise Elemente der Antriebseinrichtung, zum Beispiel eine Antriebshülse, drehbar lagern. Die Führung für das Halteelement kann beispielsweise im Gehäuse, im Gehäuseeinsatz, in sonstigen Elementen im Gehäuse oder teilweise im Gehäuse und teilweise im Gehäuseeinsatz ausgebildet sein.

Die Antriebseinrichtung dient zum Ausschütten des Produkts aus dem Produktbehälter. In einer bevorzugten Ausführung umfasst die Antriebseinrichtung eine Antriebshülse und eine Kolbenstange. Die Antriebshülse treibt die Kolbenstange an, so dass sich diese in distaler Richtung bewegt und den Stopfen im Produktbehälter verschieben kann zum Ausschütten des Produkts. Die Kupplungshülse treibt vorzugsweise die Antriebshülse an. Die Antriebshülse kann dabei nur verschiebbar, drehbar oder verschiebbar und drehbar, insbesondere mittels einer Schraubbewegung bewegbar im Gehäuse gelagert sein, um die Kolbenstange anzutreiben. Zum Ausschütten des Produkts mittels einer Schraubenbewegung bewegbar oder nur verschiebbar im Gehäuse gelagert sein.

In einer bevorzugten Ausführung ist die Antriebshülse rotationsfest aber axial verschiebbar relativ zur Kolbenstange gehalten, wobei die Kolbenstange innerhalb der Antriebshülse angeordnet ist. Die Antriebshülse ist dabei relativ zum Gehäuse rotierbar aber in Längsrichtung des Gehäuses relativ zum Gehäuse nicht verschiebbar gelagert.

Die Kupplungshülse ist beim Einstellen, Korrigieren und Ausschütten der Dosis vorzugsweise in axialer Richtung relativ zum Gehäuse und relativ zu einem allenfalls vorhandenen Gehäuseeinsatz verschiebbar. Ferner ist die Kupplungshülse wahlweise mittels des Halteelements relativ zum Gehäuse rotativ fixierbar oder rotativ frei. Weiter ist die Kupplungshülse innerhalb des Gehäuses angeordnet und umgibt vorzugsweise die Elemente der Antriebsvorrichtung, insbesondere eine Antriebshülse. Dabei ist die Kupplungshülse in einer bevorzugten Ausführung in Längsrichtung des Gehäuses relativ zur Antriebseinrichtung verschiebbar. Dadurch kann die Kupplungshülse an unterschiedlichen axialen Positionen mit der Antriebseinrichtung zusammenwirken.

Wird der Ausschüttvorgang ausgelöst, beispielsweise indem direkt oder indirekt über ein weiteres Element die Kupplungshülse relativ zum Halteelement bewegt wird, kann die Kupplungshülse vom Halteelement freigegeben werden. Die Kupplungshülse kann dann, beispielsweise mittels einer Rotationsbewegung oder Schraubbewegung wieder in das Gehäuse hineingeführt werden und durch die Rotation oder Verschiebung relativ zum Gehäuse mit der Antriebseinrichtung zusammenwirken, so dass das Produkt ausgeschüttet werden kann.

Das Halteelement ist in der Führung im Gehäuse, einem allenfalls vorhandenen Gehäuseeinsatz oder in einem sonstigen Element innerhalb des Gehäuses in Richtung der Längsachse des Gehäuses relativ zum Gehäuse verschiebbar jedoch stets rotationsfest relativ zum Gehäuse geführt. Dabei ist das Halteelement bevorzugt sowohl beim Einstellen und Korrigieren wie auch beim Ausschütten einer eingestellten Dosis in Richtung Längsachse verschiebbar. Weiter ist das Halteelement durch die Führung oder mittels weiteren Elementen rotationsfest relativ zum Gehäuse gehalten. Das ist beispielsweise mit einem Zahn, Nocken oder Steg im Halteelement und einer gegenförmigen in axialer Richtung erstreckende Nut oder Ausformung im Gehäuse oder im Gehäuseeinsatz ermöglicht. Alternativ kann jedoch das Gehäuse, ein Gehäuseeinsatz oder ein sonstiges gehäusefestes Element einen Zahn, Nocken oder Steg umfassen und das Halteelement eine entsprechend geformte Nut oder Ausformung aufweisen.

In einer bevorzugten Ausführung ist die Kupplungshülse beim Einstellen und Korrigieren einer Dosis mittels Verzahnung zwischen Kupplungshülse und Halteelement rotationsfest relativ zum Gehäuse gehalten. Zum Ausschütten der Dosis kann die Kupplungshülse bevorzugt relativ zum Halteelement in Richtung der Längsachse verschoben werden, so dass die Verzahnung zwischen Kupplungshülse und Halteelement aufgehoben ist und die Kupplungshülse relativ zum Gehäuse rotieren kann.

Das Halteelement hält beim Einstellen und Korrigieren der Dosis die Kupplungshülse, vorzugsweise mittels eines Eingriffelements, einer Verzahnung oder mittels einer kraftschlüssigen Verbindung. Beim Ausschütten hingegen kann die Kupplungshülse vom Halteelement freigegeben werden. Das bedeutet, die Kupplungshülse ist nicht länge am Halteelement gehalten, so dass die Kupplungshülse sich relativ zum Gehäuse bewegen kann. Beispielsweise ist das Eingriffselement nicht mehr im Eingriff oder die kraftschlüssige Verbindung ist aufgehoben.

Bevorzugt weist die Kupplungshülse ein Eingriffselement und das Halteelement ein Gegeneingriffselement auf, wobei das Eingriffselement und das Gegeneingriffselement ineinander eingreifen können zum rotativen Koppeln der Kupplungshülse mit dem Haltelement. Dadurch ist bei Eingriff die Kupplungshülse auch rotationsfest relativ zum Gehäuse gehalten.

Das Eingriffselement und das Gegeneingriffselement können beispielsweise jeweils aus mindestens zwei Zähne gebildet werden, welche ineinander greifen können. Eingriffselement und Gegeneingriffselement können aber auch als Auskragung (Nocken, Steg, Rippe) und eine die Auskragung aufnehmende Nut realisiert sein. Dabei weist die Kupplungshülse eine aus Auskragung oder Nut auf und das Halteelement weist die andere aus Auskragung oder Nut auf.

Das Dosiseinstellelement ist in Richtung Längsachse relativ zum Gehäuse bewegbar. Bevorzugt ist das Dosiseinstellelement zudem drehbar relativ zum Gehäuse oder drehbar und verschiebbar, insbesondere mittels einer Schraubenbewegung relativ zum Gehäuse bewegbar. Das Dosiseinstellelement kann beispielsweise ringförmig, hülsenförmig oder schalenförmig ausgebildet sein. Weiter kann das Dosiseinstellelement aber auch als axialer Schieber ausgeführt sein, um die Kupplungshülse in Abhängigkeit der Dosis in axialer Richtung zu positionieren. Ferner kann das Dosiseinstellelement als drehbarer und axial verschiebbarer Griff, als Hülse oder als Knopf ausgeführt sein. In einer bevorzugten Ausführung ist das Dosiseinstellelement als Dosierhülse ausgebildet, welche koaxial zur Längsachse des Gehäuses im Gehäuse angeordnet ist.

In einer bevorzugten Ausführung sind zum Einstellen und Korrigieren einer Dosis das Dosiseinstellelement und die Kupplungshülse voneinander entkoppelt, so dass sich das Dosiseinstellelement relativ zur Kupplungshülse bewegen kann. Zum Ausschütten der Dosis sind in dieser Ausführung vorzugsweise das Dosiseinstellelement und die Kupplungshülse miteinander gekoppelt, so dass das Dosiseinstellelement nicht relativ zur Kupplungshülse bewegbar ist.

Bevorzugt wird beim Wechsel vom Einstellen und Korrigieren der Dosis (a) zum Ausschütten der Dosis (b) die Kupplungshülse vom Halteelement freigegeben und anschliessend nach der Freigabe der Kupplungshülse wird diese mit dem Dosiseinstellelement gekoppelt. In einer weiteren Ausführung besteht jedoch auch die Möglichkeit, dass die Kupplungshülse vom Halteelement freigegeben wird und gleichzeitig mit dem Dosiseinstellelement gekoppelt wird. In einer dritten Ausführung kann jedoch auch zuerst die mittels des Halteelements rotationsfest zum Gehäuse gehaltene Kupplungshülse zusätzlich mit dem Dosiseinstellelement gekoppelt werden und erst anschliessend die Kupplungshülse vom Halteelement freigegeben werden.

Der Begriff "distal" bezeichnet eine zum vorderen, einstechseitigen Ende der Injektionsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Injektionsvorrichtung hin gerichtete Seite oder Richtung.

Der Begriff axial bezieht sich auf die Längsachse des Gehäuses. Entsprechend ist eine axiale Richtung parallel zur Längsachse des Gehäuses oder in Längsrichtung des Gehäuses. Eine radiale Richtung bezieht sich auf eine Richtung senkrecht zur Längsachse des Gehäuses.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionsvorrichtung" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

Vorzugsweise kann zum Ausschütten durch Verschieben der Kupplungshülse relativ zum Halteelement in Richtung der Längsachse des Gehäuses die Kupplungshülse vom Halteelement rotativ freigegeben werden, wodurch die Kupplungshülse relativ zum Gehäuse rotieren kann. Vorzugsweise kann durch die rotierende Kupplungshülse die Antriebseinrichtung angetrieben und die eingestellte Dosis ausschüttet werden. Unter dem Begriff "Verschieben" wird eine Bewegung entlang eines linearen, gradlinigen oder gekrümmten, jedoch nicht kreisrunden Weges verstanden. Demnach unterscheidet sich eine Verschiebung von einer Drehung. Bei Letzterer erfolgt die Bewegung entlang eines Kreises. Unter "linearem Weg" wiederum wird eine Linie verstanden, welche stetig differenzierbar ist.

Durch die Verschiebbarkeit der Kupplungshülse kann diese rasch und unkompliziert vom Halteelement entkoppelt werden, beispielsweise wenn der Benutzer durch Betätigen eines Druckknopfs die Ausschüttung der Dosis aktiviert.

Alternativ besteht auch die Möglichkeit, dass die Kupplungshülse mittels Drehung relativ zum Halteelement von diesem freigegeben wird oder indem zum Beispiel radiale Eingriffselement der Kupplungshülse in radialer Richtung aus einem Eingriff mit dem Halteelement gelöst werden, so dass die Kupplungshülse relativ zum Halteelement rotierbar ist.

Bevorzugt ist durch die Verschiebung der Kupplungshülse zudem die Kupplungshülse rotationsfest mit dem Dosiseinstellelement koppelbar. Dabei bedeutet "koppelbar", dass die Kupplungshülse lösbar mit dem Dosiseinstellelement verbunden ist. Die Kopplung ist also jederzeit wieder auflösbar, indem die Kupplungshülse vom Dosiseinstellelement entkoppelt wird, so dass sich die Kupplungshülse wieder relativ zum Dosiseinstellelement drehen kann.

Die relative Verschiebung der Kupplungshülse zum Halteelement bewirkt also einerseits die rotative Freigabe (oder Entkopplung) der Kupplungshülse vom Halteelement und andererseits die rotative Kopplung der Kupplungshülse mit dem Dosiseinstellelement. Nach der Verschiebung ist die Kupplungshülse somit relativ zum Halteelement drehbar aber rotationsfest relativ zum Dosiseinstellelement gehalten. Somit sind mit einer einzigen Verschiebebewegung der Kupplungshülse zwei Kupplungen schaltbar.

Nach erfolgter Kopplung der Kupplungshülse mit dem Dosiseinstellelement ist vorzugsweise eine Bewegung, insbesondere eine Schraubbewegung, des Dosiseinstellelements auf die Kupplungshülse übertragbar.

Bevorzugt ist die Kupplungshülse mittels Verzahnung rotationsfest mit dem Dosiseinstellelement koppelbar. Dadurch lässt sich sicher und zuverlässig ein Drehmoment vom Dosiseinstellelement auf die Kupplungshülse und umgekehrt übertragen.

Wie erwähnt, bedeutet der Begriff "koppelbar", dass die Verbindung lösbar ist. Der Begriff "Verzahnung" ist nicht auf eine Verbindung mit zwei ineinandergreifende Zähne beschränkt, sondern umfasst jede Verbindung mit Eingriffselementen, bei der mittels Formschluss eine Kraft übertragen werden kann. So kann beispielsweise die Kupplungshülse oder das Dosiseinstellelement Eingriffselemente in der Form von Nocken, Zacken, Zinken, Auskragungen, Keilen, Stiften oder sonstigen Ausformungen aufweisen, welche in entsprechen ausgeformte Nuten, Vertiefungen, Ausnehmungen, Löcher oder Gegeneingriffselemente im Gegenstück, also im Dosiseinstellelement oder in der Kupplungshülse, eingreifen können, so dass sich Kupplungshülse und Dosiseinstellelement nicht relativ zueinander verdrehen können.

Dabei umfasst das Dosiseinstellelement vorzugsweise mindestens eine in Richtung Längsachse ausgerichtete Nut und die Kupplungshülse weist mindestens einen radialen Nocken auf, welcher in die mindestens eine Nut eingreifen kann, wodurch die Kupplungshülse einfach und sicher rotationsfest mit dem Dosiseinstellelement koppelbar ist. In einer bevorzugten Ausführung kann diese Kopplung durch relatives Verschieben der Kupplungshülse zur Dosierhülse erstellt werden, indem der mindestens eine Nocken in Richtung der Längsachse in die mindestens eine Nut eingeführt wird.

**In** einer alternativen Ausführung besteht auch die Möglichkeit, dass die Kupplungshülse mittels Kraftschluss, beispielsweise mittels Kegelverbindung (konischer Stift und keglige Bohrung) rotationsfest mit dem Dosiseinstellelement verbunden wird.

**In** einer bevorzugten Ausführung kann die Kupplungshülse mittels Verzahnung rotationsfest relativ zum Halteelement gehalten werden. Dadurch ist die Kupplungshülse sicher und zuverlässig gegen Rotation relativ zum Halteelement gehalten. Da zudem das Halteelement rotationsfest relativ zum Gehäuse gehalten ist, kann die Kupplungshülse sicher relativ zum Gehäuse rotationsfest gehalten werden.

Wie oben erwähnt, bezieht sich der Begriff "Verzahnung" nicht nur auf Zähne, sondern umfasst alle miteinander in Eingriff bringbare Eingriffselemente.

Vorzugsweise ist die Verzahnung mit mindestens einem Nocken und mindestens einer Nut ausgebildet. **In** diesem Fall weist vorzugsweise das Halteelement mindestens eine in Richtung der Längsachse des Gehäuses ausgerichtete Nut auf und die Kupplungshülse weist mindestens einen radialen Nocken oder Zahn auf, welche in die mindestens eine Nut eingreifen kann. Eine solche Nut und ein Nocken sind einfach herstellbar. Zudem ist die Kupplungshülse dadurch rasch und einfach rotationsfest mit dem Halteelement koppelbar bzw. entkoppelbar.

Falls die Kupplungshülse durch Verschieben relativ zum Halteelement vom Halteelement freigegeben werden kann, können vorzugsweise bei der Verschiebung Eingriffselement vom Halteelement in Gegeneingriffselemente in der Kupplungshülse in Eingriff gebracht werden. Entsprechend können in diesem Fall auch durch die Verschiebung die Eingriffselemente von den Gegeneingriffselementen gelöst werden, so dass die Kupplungshülse vom Halteelement freigegeben ist und relativ zum Halteelement rotieren kann.

In einer alternativen Ausführung können Kupplungshülse und Halteelement kraftschlüssig relativ zueinander gehalten werden, so dass keine Rotation zwischen Kupplungshülse oder Halteelement möglich ist.

In einer bevorzugten Ausführung ist die Führung als Nut ausgebildet und das Halteelement ist mittels einer Ausformung rotationsfest relativ zum Gehäuse in der Nut geführt. Hierzu kann das Halteelement beispielsweise ein Zahn, ein Stift oder eine sonstige Ausformung aufweisen, welche in die Nut eingreifen kann. Mit Vorteil, besteht die Führung aus mehreren Nuten und das Halteelement weist entsprechend mehrerer Ausformungen auf.

Alternativ besteht auch die Möglichkeit, dass das Halteelement eine Nut umfasst und das Gehäuse, ein Gehäuseeinsatz oder ein sonstiges gehäusefestes Element eine Ausformung aufweist, welche in die Nut eingreifen kann.

Bevorzug weist das Halteelement eine Öffnung auf, in welcher die Kupplungshülse aufgenommen werden kann. Das ermöglicht eine axial platzsparende Anordnung, da in diesem Fall das Halteelement nicht auf der distalen oder proximalen Seite der Kupplungshülse platziert werden muss. Das Halteelement ist dabei vorzugsweise hülsenförmig oder ringförmig ausgebildet und wirkt mit seiner Aussenseite mit dem Gehäuse oder einem allenfalls vorhandenen Gehäuseeinsatz zusammen. Die Innenseite der Öffnung wirkt vorzugsweise mit einer Aussenseite der Kupplungshülse zusammen.

Alternativ besteht auch die Möglichkeit, dass das Halteelement keine Öffnung aufweist und insbesondere nicht rotationssymmetrisch ausgebildet ist. So kann das Halteelement beispielsweise nur auf einer Seite der Kupplungshülse platziert sein um diese relativ zum Gehäuse zu halten.

In einer bevorzugten Ausführung ist das Halteelement scheibenförmig ausgebildet und weist im Zentrum die Öffnung auf, in der koaxial zum Halteelement die Kupplungshülse aufgenommen werden kann. Dadurch ist das Halteelement kompakt konstruierbar.

Vorzugsweise ist das Dosiseinstellelement als Dosierhülse ausgebildet, welche axial am Halteelement gehalten ist, so dass sich das Halteelement zusammen mit der Dosierhülse in Richtung der Längsachse bewegen kann.

Bevorzugt steht die Dosierhülse in Gewindeverbindung mit dem Gehäuse oder einem Gehäuseeinsatz. In diesem Fall kann durch eine Schraubbewegung die auszuschüttende Dosis eingestellt oder korrigiert werden. Ferner ist bevorzugt die Kupplungshülse koaxial in der Dosierhülse angeordnet, wodurch die Dosierhülse radial zwischen Kupplungshülse und Gehäuse oder Gehäuseeinsatz angeordnet ist.

Die Dosierhülse ist in axialer Richtung am Halteelement gehalten, vorzugsweise mit diesem verschnappt, wodurch sich das Halteelement beim Einstellen und Korrigieren wie auch beim Ausschütten axial zusammen mit der Dosierhülse relativ zum Gehäuse bewegen kann. Die Dosierhülse ist dabei jedoch drehbar relativ zum Halteelement, welches rotationsfest zum Gehäuse ist. Vorzugsweise ist das Halteelement an einer distalen Seite der Dosierhülse angeordnet.

In einer bevorzugten Ausführung umfasst die Dosiereinrichtung ein elastisches Element, welches die Kupplungshülse mit einer Vorspannkraft in einer Dosierstellung hält, in welcher die Kupplungshülse rotationsfest relativ zum Halteelement gehalten ist. Falls die Kupplungshülse mittels Verzahnung rotationsfest zum Halteelement gehalten werden kann, sind vorzugsweise in der Dosierstellung Eingriffselement der Kupplungshülse mit den Gegeneingriffselemente des Halteelements in Eingriff.

Durch die Vorspannung muss zuerst die Vorspannkraft überwunden werden, um die Kupplungshülse aus der Dosierstellung zu bewegen. Dadurch vermindert sich das Risiko, dass sich die Kupplungshülse unbeabsichtigt aus der Dosierstellung wegbewegt und die Antriebseinrichtung betätigt wird. In der Dosierstellung kann die Kupplungshülse nicht rotieren und damit nicht ungewollt die Antriebseinrichtung antreiben.

Wird entgegen der Vorspannkraft die Kupplungshülse aus der Dosierstellung in distaler Richtung relativ zum Halteelement bewegt, wird die Kupplungshülse vorzugsweise vom Halteelement freigegeben, so dass die Kupplungshülse relativ zum Halteelement und zum Gehäuse rotieren kann.

Das elastische Element lässt sich elastisch verformen und durch die Komprimierung kann die Vorspannkraft erzeugt werden. Das elastische Element kann beispielsweise eine metallische Feder, ein elastisch verformbares Metallelement oder ein elastisches Kunststoffmaterial, insbesondere ein Gummielement, sein.

Bevorzugt umfasst das elastische Element ein Grundkörper und mindestens zwei elastische Arme, wobei der Grundkörper in einer Ebene senkrecht zur Längsachse des Gehäuses ausgerichtet ist und ein erster der zwei Arme in proximale Richtung und ein zweiter der zwei Arme in distale Richtung weist. Dabei sind die Arme so am Grundkörper angebracht, dass die Arme in axialer Richtung komprimiert, ausgelenkt oder gebogen werden können. Durch eine elastische Verformung der Arme zum Grundkörper hin lässt sich das elastisch Element in der axialen Länge komprimieren. Dadurch ist die Vorspannkraft erzeugbar, mit welcher die Kupplungshülse in der Dosierstellung gehalten werden kann.

Weiter weist in einer bevorzugten Ausführung der Grundkörper eine Öffnung auf, so dass der Grundkörper ringförmig ist und beispielsweise die Kupplungshülse umgeben kann.

Zudem umfasst das Dosiseinstellelement bevorzugt Zähne und die Kupplungshülse umfasst Zähne, wobei zum Erzeugen eines akustischen oder taktilen Signals ein erster der mindestens zwei Arme mit den Zähnen des Dosiseinstellelements oder ein zweiter der mindestens zwei Arme mit Zähnen der Kupplungshülse zusammenwirken kann. Indem beispielsweise das Dosiseinstellelements relativ zum elastischen Element rotiert, wird der erste oder der zweite Arm über die Zähne des Dosiseinstellelements bewegt. Dadurch wird der Arm durch die steigende Zahnflanke immer mehr elastisch ausgelenkt bis er den höchsten Punkt des Zahns überwunden hat. Anschliessend federt der Arm zurück und erzeugt dabei ein akustisches und/oder taktiles Signal, insbesondere ein Klickgeräusch, welches der Benutzer wahrnehmen kann. In gleicher Weise kann ein akustisches und/oder taktiles Signal erzeugt werden, wenn ein Arm über die Zähne der Kupplungshülse bewegt wird. Um diese Signale besonders deutlich erzeugen zu können, sind die Zähne des Dosiseinstellelements und der Kupplungshülse bevorzugt sägezahnförmig ausgebildet.

Dabei sind die Zähne und das elastische Element vorzugsweise so angeordnet, dass in einer ersten Drehrichtung der erste Arm über die Zähne des Dosiseinstellelements bewegt wird, während jedoch der zweite Arm nicht relativ zu den Zähnen der Kupplungshülse bewegbar ist. In einer zweiten, der ersten Drehrichtung entgegengesetzten Richtung wird dann der zweite Arm über die Zähne der Kupplungshülse bewegt, während der erste Arm nicht relativ zu den Zähnen des Dosiseinstellelements bewegbar ist. Dadurch wird in beiden Drehrichtungen jeweils ein akustisches und/oder taktiles Signal erzeugt.

Vorzugsweise weisen das Dosiseinstellelement und die Kupplungshülse in einem proximalen Bereich je einen Flansch auf und das elastische Element ist in Richtung der Längsachse zwischen diesen Flanschen angeordnet. Das elastische Element wirkt somit auf eine Flanschfläche und kann dadurch die durch die Vorspannung erzeugte Kraft besonders gut auf das Dosiseinstellelement und die Kupplungshülse übertragen. Dabei ist die Flanschfläche bevorzugt rechtwinklig zur Längsachse ausgerichtet.

Indem das elastisch Element in einem proximalen Bereich angeordnet ist, kann es optimal konstruiert werden und muss nicht im Bereich des Halteelements platziert werden, wo eine platzsparende Anordnung kaum realisierbar ist.

Ferner umfasst in einer bevorzugten Ausführung die Dosiereinrichtung einen Gehäuseeinsatz, welcher eine Ausnehmung umfasst, die die Führung für das Halteelement bildet. Bevorzugt ist dabei die Ausnehmung in der Form einer Nut ausgeführt, in der das Halteelement in Längsrichtung geführt werden kann und rotationsfest relativ zum Gehäuseeinsatz gehalten ist. Vorzugsweise ist der Gehäuseeinsatz koaxial zum Gehäuse angeordnet. Dabei kann der Gehäuseeinsatz eine zylindrische, hülsenförmige oder schalenförmige Gestalt aufweisen. Der Gehäuseeinsatz ist in axialer und radialer Richtung unbeweglich relativ zum Gehäuse und an diesem fixiert, beispielsweise mittels einer Schnappverbindung.

Vorzugsweise ist der Gehäuseeinsatz hülsenförmig und koaxial zur Längsachse des Gehäuses im Innern des Gehäuses platziert. Durch den Gehäuseeinsatz ist die Führung des Halteelements unabhängig vom Gehäuse realisiert. Das Gehäuse kann daher unabhängig vom Halteelement und von der Kupplungshülse für verschiedene Ausführungen angepasst werden.

Bevorzugt weist der Gehäuseeinsatz ein Innengewinde auf, mit dem das Dosiseinstellelements in Gewindeverbindung steht, so dass das Dosiseinstellelement zum Einstellen und Korrigieren einer Dosis in den Gehäuseeinsatz ein- und ausschraubbar ist. Dabei kann der Gehäuseeinsatz ein- oder mehrteilig ausgeführt sein. Beispielsweise kann der Gehäuseeinsatz als Hülse ausgeführt sein, welche in einem distalen Bereich die Antriebseinrichtung und in einem proximalen Bereich das Dosiseinstellelement lagert. Alternativ dazu kann der Gehäuseeinsatz aber auch einen ersten Teil umfassen, welcher die Antriebseinrichtung lagert und einen zweiten Teil, welcher das Dosiseinstellelement aufnimmt.

In einer bevorzugten Ausführung weist die Antriebseinrichtung eine Antriebshülse und eine Kolbenstange auf. In diesem Fall ist bevorzugt die Antriebshülse rotierbar aber axial fest relativ zum Gehäuseeinsatz im Gehäuseeinsatz gelagert. Ferner weist der Gehäuseeinsatz bevorzugt ein zweites Innengewinde auf zum Aufnehmen der Kolbenstange. Indem der Gehäuseeinsatz die Führung für das Halteelement bildet wie auch die Dosierhülse und die Antriebseinrichtung lagert, dient das Gehäuse nur als äussere Ummantelung und hat keine weitere Funktion. Das Gehäuse ist daher unabhängig von den Elemente und Funktionen der Dosiereinrichtung und kann entsprechend unabhängig gestaltet werden, beispielsweise um bestimmte ergonomische oder ästhetische Anforderungen zu erfüllen.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt eine perspektivische Gesamtansicht der erfindungsgemässen Injektionsvorrichtung;
- Fig. 2: zeigt in perspektivischer Ansicht eine Explosionsdarstellung der Einzelteile der erfindungsgemässen Dosiereinrichtung der Injektionsvorrichtung aus Figur 1;
- Fig. 3: zeigt eine Schnittansicht der Injektionsvorrichtung in einer Ausgangsstellung, wobei der Schnitt durch die Längsachse verläuft;
- Fig. 4: zeigt eine Schnittansicht, wobei der Schnitt durch eine Ebene rechtwinklig zur Längsachse verläuft;
- Fig. 5: zeigt eine Schnittansicht durch die Längsachse, wobei eine zu verabreichende Dosis eingestellt ist;
- Fig. 6: zeigt Vergrösserung der Schnittansicht durch die Längsachse im Bereich des Halteelements, wobei in der gezeigten Stellung die Kupplungshülse vom Halteelement freigegeben ist;
- Fig. 7: zeigt eine Schnittansicht rechtwinklig zur Längsachse, wobei der Schnitt im Bereich des Halteelements durch die Injektionsvorrichtung verläuft;
- Fig. 8: zeigt eine perspektivische Darstellung der Kupplungshülse, mit Halteelement und Antriebshülse;
- Fig. 9: zeigt eine Schnittansicht durch die Längsachse nach dem Ausschütten einer Dosis;
- Fig. 10: zeigt eine perspektivische Ansicht des proximalen Endbereichs der Injektionsvorrichtung, wobei der Griff der Dosierhülse zur besseren Ansicht nur abschnittsweise dargestellt ist;
- Fig. 11: zeigt eine perspektivische Ansicht des Klickrings;
- Fig. 12: zeigt einen proximalen Endbereich einer zweiten Ausführung der erfindungsgemässen Injektionsvorrichtung, wobei der Griff der Dosierhülse nur abschnittsweise dargestellt ist;
- Fig. 13: zeigt eine Draufsicht des Klickrings der zweiten Ausführung aus Figur 12;
- Fig. 14: zeigt eine perspektivische Ansicht des Klickrings aus Figur 13;
- Fig. 15: zeigt einen proximalen Endbereich einer dritten Ausführung der erfindungsgemässen Injektionsvorrichtung, wobei der Griff der Dosierhülse nur abschnittsweise dargestellt ist;
- Fig. 16: zeigt eine perspektivische Ansicht des Klickrings der dritten Ausführung aus Figur 15;
- Fig. 17: zeigt einen proximalen Endbereich in der Schnittansicht der dritten Ausführung aus Figur 15;
- Fig. 18: zeigt eine weitere Ausführung der Antriebshülse;
- Fig. 19: zeigt eine Schnittansicht der Antriebshülse aus Fig. 18 zusammen mit der Dosierhülse, wobei der Schnitt rechtwinklig zur Längsachse verläuft;
- Fig. 20: zeigt eine perspektivische Ansicht einer weiteren Ausführung der Dosierhülse und
- Fig. 21: zeigt eine perspektivische Ansicht einer weiteren Ausführung des Gehäuseeinsatzes;
- Fig. 22: zeigt eine perspektivische Ansicht einer weiteren Ausführung des Halteelements und der Kupplungshülse beim Einstellen und Korrigieren einer Dosis;
- Fig. 23: zeigt eine Schnittansicht der Ausführung aus Fig. 22, wobei der Schnitt senkrecht zur Längsachse durch die Ratschenarme verläuft;
- Fig. 24: zeigt die Ausführung aus Figur 22 beim Ausschütten der Dosis;
- Fig. 25: zeigt eine Schnittansicht der Ausführung aus Figur 24 beim Ausschütten.

### FIGURENBESCHREIBUNG

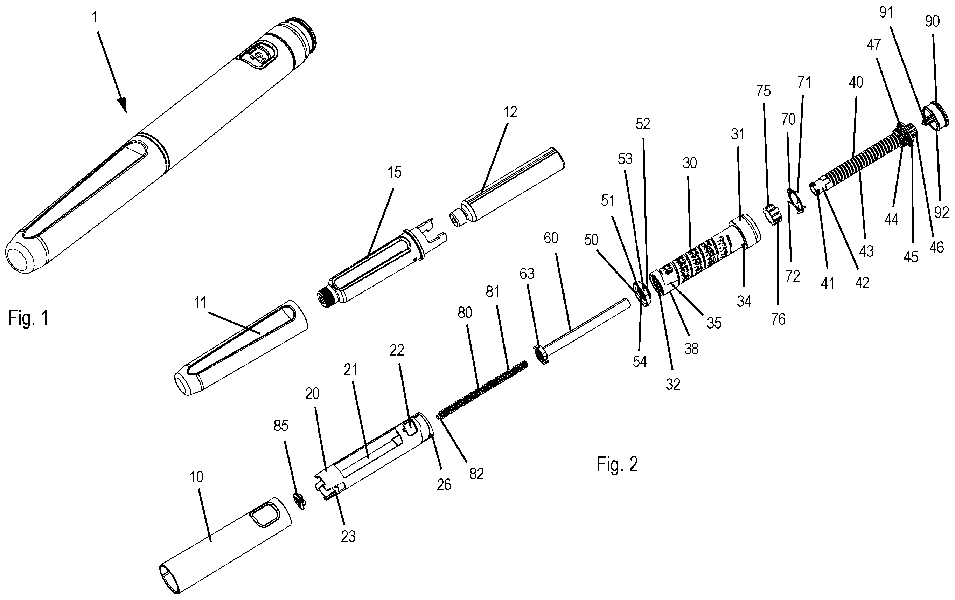

Figur 1 zeigt eine perspektivische Ansicht einer erfindungsgemässen Injektionsvorrichtung in der Form eines Injektors 1, welcher die erfindungsgemässe Dosiereinrichtung umfasst. Figur 2 zeigt die Einzelteile des Injektors 1 mit der Dosiereinrichtung in einer Explosionsdarstellung. Dabei befindet sich das distale, einstechseitige Ende des Injektors 1 im linken Bereich der Figur 2 und das proximale Ende des Injektors 1 im rechten, oberen Bereich der Figur 2.

Der Injektor 1 ist in der gezeigten Ausführung als Einweginjektor ausgebildet. Wie in Figur 2 ersichtlich, umfasst der Injektor 1 eine abnehmbare Schutzkappe 11, ein längliches, zylinderförmiges Gehäuse 10 in dem sich ein Gehäuseeinsatz 20 befindet, ein Karpulenhalter 15 in dem eine Karpule 12 mit einer medizinischen Substanz gehalten ist, ein als Dosierhülse 30 zum Einstellen einer Dosis ausgebildetes Dosiseinstellelement, ein mit der Dosierhülse 30 koppelbares Halteelement 50, eine Antriebshülse 60, eine Kupplungshülse 40 zum Koppeln der Dosierhülse 30 mit der Antriebshülse 60 und eine Kolbenstange 80, welche durch die Antriebshülse 60 angetrieben wird zum Ausschütten der medizinischen Substanz aus der Karpule 12.

Nachfolgend wird auf die strukturellen Merkmale der einzelnen Bestandteile des Injektors 1 im Detail eingegangen. Die Funktion, insbesondere das Einstellen, Korrigieren und Ausschütten einer Dosis, ist im Anschluss beschrieben.

Der Karpulenhalter 15 ist an einem distalen Ende des Gehäuses 10 mittels einer Schnappverbindung rotationsfest und axialfest mit dem Gehäuse 10 verschnappt. Der Karpulenhalter 15 lagert die Karpule 12 und weist an seinem distalen Ende ein Verbindungselement auf, an dem eine Injektionsnadel (nicht dargestellt) angebracht werden kann.

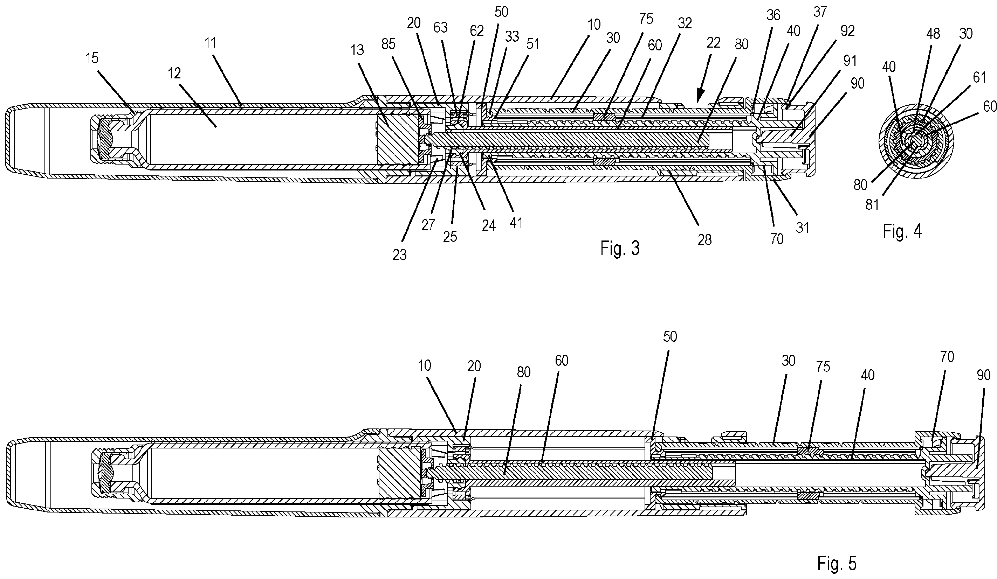

Der Gehäuseeinsatz 20 hat eine zylindrische Form und ist koaxial im Gehäuse 10 angeordnet. Über Nocken 26 an seiner Aussenseite, welche in entsprechende Ausnehmungen auf der Innenseite des Gehäuses 10 eingreifen ist der Gehäuseeinsatz 20 sowohl in axialer Richtung wie auch rotativ unbeweglich relativ zum Gehäuse 10 an diesem verschnappt. In einem distalen Bereich weist der Gehäuseeinsatz 20 in seinem Innern ein erstes Innengewinde 27 auf, in welchem die Kolbenstange 80 eingeschraubt ist, ersichtlich in Figur 3. Ebenfalls im distalen Bereich im Innern des Gehäuseeinsatzes 20 weist dieser eine zylinderförmige Aufnahme mit einer umlaufenden Wulst 24 auf, wobei dieser Wulst 24 eine axiale Halterung für die Antriebshülse 60 bildet, erkennbar in Figur 3.

Im proximalen Bereich weist der Gehäuseeinsatz 20 ein zweites Innengewinde 28 auf, in welches die Dosierhülse 30 eingeschraubt ist. Im Bereich dieses zweiten Innengewindes 28 ist ein radialer Durchbruch oder eine Öffnung 22 (erkennbar in Figur 2) im Mantel des Gehäuseeinsatzes 20 ausgeformt. Ebenso weist das Gehäuse 10 an dieser Stelle in radialer Richtung eine Öffnung auf. Dadurch ist von aussen eine auf der eingeschraubten Dosierhülse 30 aufgedruckte Skala ablesbar. Der Gehäuseeinsatz 20 lagert somit Kolbenstange 80, Antriebshülse 60 und Dosierhülse 30 drehbar relativ zum Gehäuse 10.

Axial in einem mittleren Bereich des Gehäuseeinsatzes 20 befinden sich axial längliche Ausnehmungen 21 im Mantel des Gehäuseeinsatzes 20, welche in Umfangrichtung 180° versetzt zueinander angeordnet sind, siehe Figur 2. Durch diese Ausnehmungen 21, welche im eingebauten Zustand des Gehäuseeinsatzes 20 eine Nut bilden, ist das Halteelement 50 in axialer Richtung im Gehäuse 10 geführt und rotationsfest am Gehäuse 10 gehalten, wie weiter unten im Detail beschrieben.

Ferner weist der Gehäuseeinsatz 20 auf der Innenseite im distalen Bereich über den Umfang verteilt radial zum Zentrum hin ragende Rippen 23 auf. Die Rippen 23 weisen in axialer Richtung gesehen eine Steigung auf, wobei die Steigung in proximaler Richtung zunimmt. Mit anderen Worten ausgedrückt, sind die Rippen 23 axial keilförmig ausgebildet und weisen distal eine kleinere radiale Höhe auf als proximal, wobei die radiale Höhe stetig Richtung proximal zunimmt (Steigung). Die Rippen 23 sind dabei so dimensioniert, dass sie plastisch verformbar sind. Die Karpule 12 wird zuerst in den Karpulenhalter 15 eingeführt. Anschliessend wird der Karpulenhalter 15 mit dem Gehäuseeinsatz 20 mit einer Schnappverbindung verbunden. Beim Zusammenführen des Karpulenhalters 15 mit dem Gehäuseeinsatz 20 berührt zuerst die im Karpulenhalter befindliche Karpule 12 die Rippen 23 des Gehäuseeinsatzes 20 an der zum Zentrum weisenden Kante. Wird der Karpulenhalter 15 weiter in proximale Richtung in den Gehäuseeinsatz 20 hineingeschoben, deformieren sich die Rippen 23 plastisch. Das heisst, die Rippen 23 werden durch die Karpule 12 bleibend verformt. Dabei werden die Rippen 23 entweder als Ganzes aus ihrer ursprünglichen Position zur Seite gedrückt oder die Rippen 23 nehmen zumindest teilweise die Form der Aussenkontur der Karpule 12 an. Wenn der Karpulenhalter 15 nun mit dem Gehäuse 10 verschnappt wird, ist die Karpule 12 axial wie auch radial unbeweglich im Karpule 15 gehalten, da der Karpulenhalter 15 eine Spannkraft in proximaler Richtung auf die Karpule 12 ausübt und diese dadurch auf die verformten Rippen 23 drückt.

Die Dosierhülse 30 steht, wie erwähnt, in Gewindeverbindung mit dem Gehäuseeinsatz 20. Hierzu weist die Dosierhülse 30 auf ihrer Aussenseite eine schraubenförmige Nut auf, welche ein Aussengewinde bildet, das mit dem Innengewinde 28 im Gehäuseeinsatz 20 zusammenwirkt. Die Dosierhülse 30 hat die Form eines Hohlzylinders oder einer Hülse und weist am proximalen Abschluss einen Bereich mit einem Durchmesser auf, welcher grösser ist als der übrige Bereich der Dosierhülse und welcher als Griff 31 dient. Dieser Griff 31 passt nicht in das Gehäuse 10, sondern steht, wie in Figur 3 ersichtlich, am proximalen Abschluss des Gehäuses 10 an. Am proximalen Ende des Bereichs mit dem kleineren Durchmesser umfasst die Dosierhülse 30 einen axial ausgerichteten Steg 34 auf der Aussenseite, erkennbar in Figur 2. Der Steg 34 schlägt in der vollständig eingeschraubten Position der Dosierhülse 30 an einen radial ausgeformten Anschlag im Gehäuseeinsatz 20 an (nicht dargestellt), so dass die minimale Dosis bzw. die Schraubbewegung der Dosierhülse 30 in das Gehäuse hinein durch den Steg 34 begrenzt wird.

An ihrem distalen Ende ist die Dosierhülse zudem auf der Aussenseite abgesetzt, so dass auf der Aussenseite ein Absatz 35 entsteht. Alternativ kann auch eine Stopphülse 38 am distalen Ende der Dosierhülse 30 auf die Dosierhülse 30 aufgeschoben sein und mit dieser rotationsfest und axialfest verbunden sein, wie in Figur 2 ersichtlich. In diesem Fall bildet eine Kante der Stopphülse 38 den Absatz 35. Dieser dient als Maximalanschlag. Wenn die Dosierhülse 30 maximal aus dem Gehäuse 10 herausgeschraubt ist, kommt der Absatz 35 mit einem Anschlag im Gehäuseeinsatz 20 in Berührung (nicht dargestellt). Dadurch wird das Herausschrauben der Dosierhülse 30 aus dem Gehäuse 10 begrenzt, so dass nicht mehr als die maximal ausschüttbare Dosis eingestellt werden kann. In einer alternativen Ausführung kann die Dosierhülse 30 im distalen Bereich auch einen axial ausgerichteten Steg umfassen, welcher in der Endposition an einem Anschlag im Gehäuseeinsatz 20 anschlägt.

Im Bereich des kleineren Durchmessers sind auf der Innenseite des Hohlzylinders der Dosierhülse 30 axiale Nuten 32 ausgebildet, in welchen eine Stoppmutter 75 axial und rotationsfest geführt wird. Hierzu weist die Stoppmutter 75 an ihrem äusseren Umfang axiale Stege 76 auf, die in die Nuten 32 ragen. Am ihrem distalen Abschluss umfasst die Dosierhülse 30 auf der Innenseite ein radial zum Zentrum hin ragender umlaufender Kragen 33, erkennbar in Figur 3. Über diesen ist ein zylinderförmiger Bereich des Halteelements 50 mit einer umlaufenden Nut 51 eingeschnappt. Das Halteelement 50 ist dadurch rotativ frei aber axial fest mit der Dosierhülse 30 verbunden.

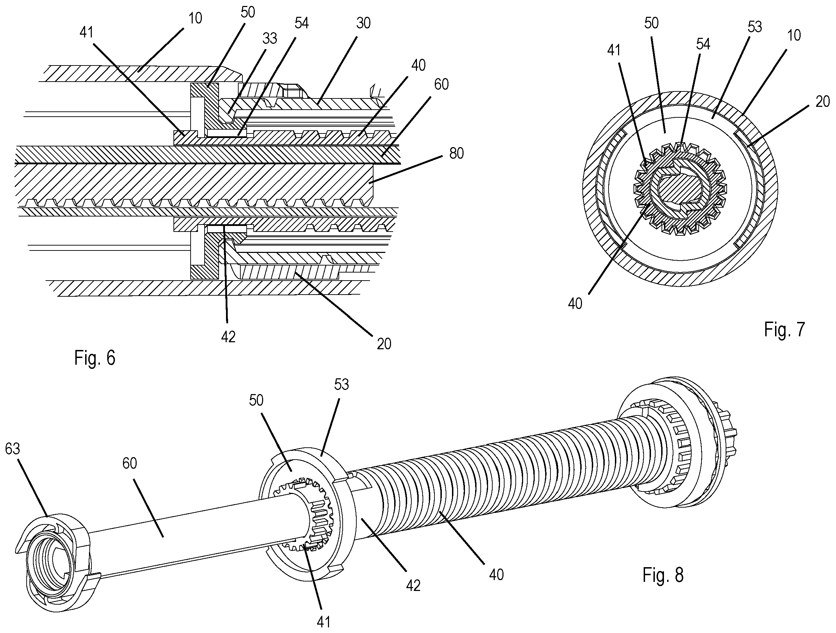

Das Halteelement 50 ist scheibenförmig und weist, wie in Figur 2 ersichtlich, in axialer Richtung gesehen einen ersten distalen Abschnitt 51 mit einem grossen Aussendurchmesser und einen zweiten proximalen Abschnitt 52 mit einem kleineren Aussendurchmesser auf, welcher in die Dosierhülse 30 einführbar ist. Das Halteelement 50 ist mit dem proximalen Abschnitt 52 mit der Dosierhülse 30 verschnappt, wie oben beschrieben. Auf der Aussenseite des distalen Abschnitts 51 sind zwei radiale Ausformungen 53 oder Nocken ausgebildet, welche sich in Umfangrichtung abschnittsweise erstrecken und zueinander um 180° versetzt angeordnet sind. Diese Ausformungen 53 werden in den länglichen Ausnehmungen 21 im Gehäuseeinsatz 20 aufgenommen und von diesen in axialer Richtung geführt. Dadurch ist das Halteelement 50 axial geführt und rotationsfest relativ zum Gehäuseeinsatz 20 und zum Gehäuse 10. Weiter weist das Halteelement 50 in seinem Zentrum eine durchgehende axiale Öffnung auf. Auf der Innenseite der Öffnung befinden sich mehrere über den Umfang verteilte axiale Nuten 54. In diese Nuten 54 des Halteelements 50 können über den Umfang verteilte im distalen Endbereich der Kupplungshülse 40 angeordnete Zähne 41 eingreifen. Dies ist in der Figur 7 erkennbar.

Die zylinderförmige, längliche Kupplungshülse 40 ist auf der proximalen Seite der Zähne 41 abgesetzt und weist einen flächigen zylinderförmigen Abschnitt 42 auf (Figur 2), dessen Aussendurchmesser kleiner ist als der innerste Innendurchmesser des Halteelements 50, so dass das Halteelement 50 den zylindrischen Abschnitt 42 nicht berührt, wenn das Halteelement 50 in axialer Richtung über diesem Abschnitt 42 positioniert ist. Auf der proximalen Seite des zylindrischen Abschnitts 42 beginnt das Aussengewinde 43 der Kupplungshülse 40, welches sich bis zu einem proximalen Endbereich der Kupplungshülse 40 erstreckt. Mit dem Aussengewinde 43 steht die Stoppmutter 75 in Gewindeeingriff. Wie in Figur 2 erkennbar, weist am proximalen Ende die Kupplungshülse 40 einen Flansch 45 sowie einen hohlzylinderförmigen Endabschnitt 46 auf, welcher einen kleineren Durchmesser hat als die übrigen Abschnitte der Kupplungshülse 40. Der Flansch 45 ist scheiben- oder schildförmig und schliesst an einen zylindrischen Abschnitt an, welcher einen grösseren Durchmesser aufweist als das Aussengewinde. Dieser Abschnitt, welcher sich auf der distalen Seite des Flansches 45 befindet, weist über den Umfange angeordnete Verbindungsstege 44 auf, die in die Nuten 32 in der Dosierhülse 30 eingeführt werden können, so dass die Kupplungshülse 40 rotationsfest mit der Dosierhülse 30 gekoppelt ist. Der scheibenförmige Flansch 45 weist auf einer distalen Flanschfläche, welche rechtwinklig zur Längsachse des Injektors 1 steht, in Umfangrichtung angeordnete Sägezähne 47 auf, siehe Figur 2.

Ist die Kupplungshülse in die Dosierhülse 30 eingeführt, wie in der Ausgangsstellung in Figur 3 gezeigt, besteht zwischen dem scheibenförmigen Flansch 45 der Kupplungshülse 40 und einer stirnseitigen Abschlusswandung oder einem Flansch der Dosierhülse 30, welche stirnseitig ebenfalls Sägezähne 36 aufweist, ein Innenraum. Der Innenraum ist in axialer Richtung etwas länger als die Verbindungsstege auf der Kupplungshülse 40, so dass diese in einer Dosierstellung der Kupplungshülse 40 nicht in die Nuten 32 der Dosierhülse 30 eingreifen, wodurch die Kupplungshülse 40 rotativ frei ist gegenüber der Dosierhülse 30.

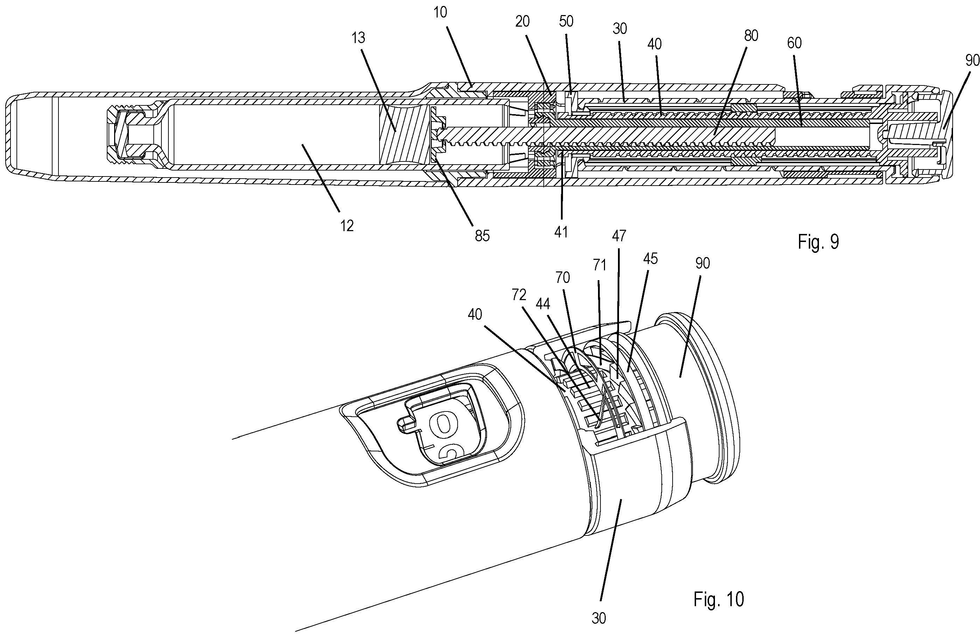

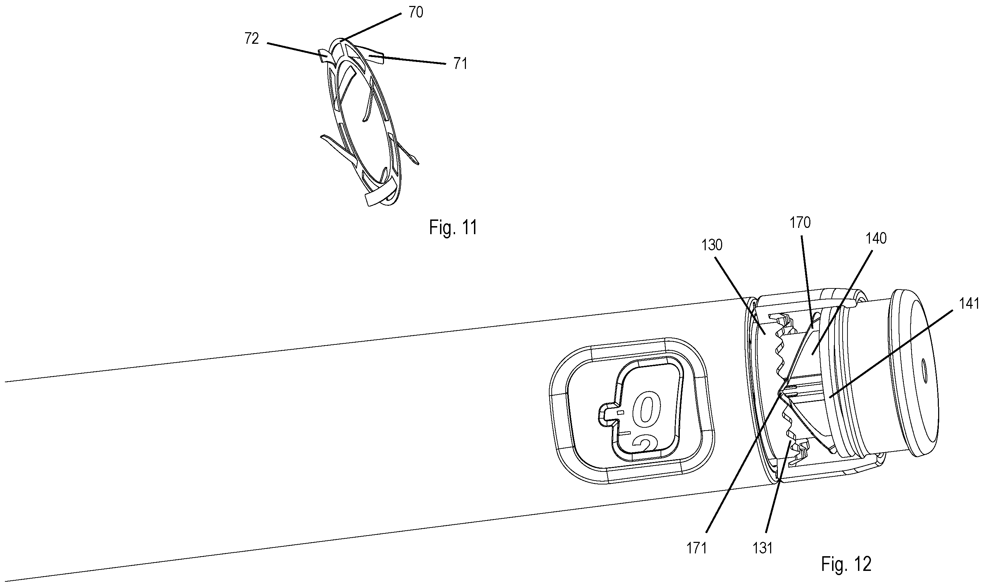

In den Figuren 10 und 11 ist der Klickring 70 ersichtlich, welcher als elastisches Element dient. Im Gegensatz zu den übrigen Bauteilen des Injektors 1, welche vorzugsweise aus Kunststoff gefertigt sind, ist die Klickscheibe 70 in einer bevorzugten Ausführung aus einem Metallblech gefertigt. Dieses ist, wie am besten in Figur 11 ersichtlich, kreisrund und hat in der Mitte eine Öffnung, so dass die Klickscheibe 70 die Form eines Rings aufweist. Dabei umfasst dieser Ring ausgestanzte distale und proximale Arme 71, 72. Das bedeutet, die Arme 71, 72 bestehen aus dem Material des Rings und sind an einer Seite mit diesem verbunden und weisen ein freies Ende auf, welches vom Ring absteht, wodurch die Arme 71, 72 ein federndes und elastisches Element bilden. Vorzugsweise umfasst der Ring auf jeder Seite vier Arme 71, 72, wobei vier distale Arme 72 in distaler Richtung und vier proximale Arme 71 in proximaler Richtung vom Ring abstehen.

Die distalen Arme 72 können dabei mit den Sägezähnen 36 der Dosierhülse 30 zusammenwirken, während die proximalen Arme 71 mit den Sägezähnen 47 der Kupplungshülse 40 zusammenwirken können, siehe Figur 10.

Wie erwähnt, ist also die Klickscheibe 70 axial zwischen der Dosierhülse 30 und der Kupplungshülse angeordnet. Dabei sind die federnden und elastischen Armen leicht vorgespannt. Durch die vom Ring abstehenden Arme werden die Dosierhülse und die Kupplungsscheibe axial auseinandergedrückt und an einen Anschlag gedrückt, welcher durch den Ausschüttknopf gebildet wird und einen maximalen axialen Abstand zwischen der Dosierhülse und der Kupplungshülse vorgibt.

In den Figuren 2 und 3 ist der Auslöseknopf 90 ersichtlich, welcher sich an einem proximalen Ende des Injektors 1 befindet. Dieser umfasst einen äusseren Hohlzylinder, welcher durch proximale Wandung abgeschlossen wird und einen im Zentrum des äusseren Hohlzylinders platzierte axiale stiftförmige Ausformung 91. Diese befindet sich im hohlzylinderförmigen Endabschnitt 46 der Kupplungshülse 40. Mit einer distalen Spitze der Ausformung 91 wird der Ausschüttknopf 90 auf der Kupplungshülse 40 abgestützt, so dass der Ausschüttknopf 90 drehbar ist relativ zur Kupplungshülse 40. Der äussere Hohlzylinder hingegen ist axial mit der Dosierhülse 30 verbunden, indem ein umlaufender Kragen 92 des Ausschüttknopfs 90 über eine umlaufende Wulst 37 am proximalen Abschluss der Dosierhülse 30 greift, dargestellt in Figur 3. Dadurch kann sich der Auslösekopf 90 nicht axial in proximaler Richtung von der Dosierhülse 30 wegbewegen. Der Ausschüttknopf 90 ist jedoch drehbar relativ zur Dosierhülse 30 und zur Kupplungshülse 40 gehalten.

Wie erwähnt, ist auf dem Aussengewinde 43 der Kupplungshülse 40 eine Stoppmutter 75 aufgeschraubt. Diese hat auf ihrer Aussenseite axiale ausgerichtete Stege 76, welche in die axialen Nuten 32 auf der Innenseite der Dosierhülse 30 eingreifen. Die Stoppmutter 75 ist demnach drehbar relativ zur Kupplungshülse 40 und axial verschiebbar aber rotationsfest relativ zur Dosierhülse 30.

In den Hohlzylinder der Kupplungshülse 40 ist die Antriebshülse 60 eingeführt. Wie in Figur 4 erkennbar, weist die Kupplungshülse 40 in ihrer axialen durchgehenden Öffnung über die ganze axiale Länge zwei in Umfangrichtung versetzte axiale Absätze oder Stege 61 auf, die im Querschnitt die Form eines Keils haben. Diese Stege 48 können in entsprechend ausgeformten axiale Nuten auf der Aussenseite der Antriebshülse 60 aufgenommen werden, wodurch die Antriebshülse 60 rotationsfest mit der Kupplungshülse 40, jedoch axial verschiebbar zur Kupplungshülse 40 in dieser gelagert ist. Dabei sind die Stege 48 so angeordnet, dass in der Drehrichtung zum Ausschütten die steile bzw. radiale Flanke des keilförmigen Stegs 48 an die entsprechend geformte radiale Flanke der Nut anliegt, so dass in Drehrichtung zum Ausschütten ein Drehmoment von der Kupplungshülse 40 optimal auf die Antriebshülse 60 übertragen werden kann.

An ihrem distalen Ende weist die Antriebshülse 60 einen Abschnitt mit einem grösseren Durchmesser auf, welcher im Innern, wie oben erwähnt, eine umlaufende Nut 62 hat, die über mit einem umlaufenden Wulst 24 des Gehäuseeinsatzes 20 verschnappt ist, so dass die Antriebshülse 60 axial relativ zum Gehäuseeinsatz 20 und dadurch zum Gehäuse 10 gehalten ist, jedoch relativ zum Gehäuseeinsatz 20 und Gehäuse 10 drehbar gelagert ist, dargestellt in Figur 3. Zudem weist die Antriebshülse 60 in diesem distalen Abschnitt zwei radial abstehende, flexible Ratschenarme 63 auf, gut ersichtlich in Figur 8. Diese wirken mit Sägezähnen 25 zusammen, welche entlang des Umfangs eines axialen Durchgangs im Gehäuseeinsatz 20 angeordnet sind (Figur 3). Die Antriebshülse 60 lässt sich dadurch nur in eine Richtung drehen, in der die Ratschenarme 63 über die flachen Flanken der Sägezähne 25 gleiten können. In der Gegenrichtung stossen die Ratschenarme 63 an steilen oder radialen Flanken der Sägezähne 25 an und verhindern dadurch eine Drehung der Antriebshülse 60 relativ zum Gehäuseeinsatz 20 und damit zum Gehäuse 10.

Ferner weist die Antriebshülse 60 im Zentrum eine kreisrunde Öffnung über ihre gesamte axiale Länge auf, wobei sich in Umfangrichtung 180° versetzt axial ausgerichtete Stege 61 befinden, welche im Querschnitt keilförmig sind, wie die Stege 48 im Innern der Kupplungshülse 40, siehe Figur 4.

In Figur 4 ist zudem erkennbar, dass die Kolbenstange 80 über ihre axiale Länge Ausformungen hat, welche einen Absatz 81 bilden. Die Kolbenstange 80 weist somit einen Querschnitt auf, welcher formschlüssig in die Öffnung der Antriebshülse 60 passt, so dass die Kolbenstange 80 rotationsfest aber axial verschiebbar relativ zur Antriebshülse 60 in dieser gelagert ist. Am distalen Ende der Kolbenstange 80 befindet sich ein knopfförmiger Abschluss 82 der Kolbenstange 80 (Figur 2), der eine Schnappverbindung mit einem Flansch 85 ermöglicht, wodurch der Flansch 85 relativ zur Kolbenstange drehbar ist, jedoch in axialer Richtung an der Kolbenstange 80 unbeweglich gehalten ist. Der Flansch 85 kann auf einen Stopfen 13 (Figur 3) in der Karpule 12 einwirken, um die medizinische Substanz aus der Karpule 12 auszuschütten.

In der Figur 3 ist der Injektor 1 in einer Ausgangsstellung gezeigt. Zum Einstellen einer Dosis wird die Dosierhülse 30 an ihrem Griff 31 relativ zum Gehäuse 10 gedreht. Da sie in Gewindeeingriff mit dem Gehäuseeinsatz 20 steht, wird sie dadurch aus dem Gehäuseeinsatz 20 herausgeschraubt. Die auf der Dosierhülse 30 aufgedruckte Zahlenskala ist durch die Öffnungen 22 im Gehäuseeinsatz 20 und im Gehäuse 10 ersichtlich und hilft beim Einstellen der gewünschten Dosis. Durch die Schraubbewegung der Dosierhülse 30 wird diese relativ zur Klickscheibe 70 rotiert. Dadurch gleiten die distalen Arme 72 der Klickscheibe 70 über die flachen Flanken der Sägezähne 36 der Dosierhülse 30 und erzeugen dabei ein Klickgeräusch. Die Klickscheibe 70 und die Kupplungshülse 40 werden nicht gedreht. Jedoch werden Klickscheibe 70 und Kupplungshülse 40 durch das Herausschrauben der Dosierhülse 30 aus dem Gehäuseeinsatz 20 axial mitgenommen und somit axial aus dem Gehäuseeinsatz (und dem Gehäuse 10) herausbewegt. Die Kupplungshülse 40 ist über die Verzahnung mit dem Halteelement 50 relativ zum Gehäuseeinsatz 20 und relativ zum Gehäuse 10 rotationsfest gehalten, da das Halteelement 50 seinerseits rotationsfest am Gehäuseeinsatz 20 und somit am Gehäuse 10 gehalten ist. Der Injektor mit einer eingestellten Dosis ist in der Figur 5 dargestellt.

Wird versehentlich eine zu hohe Dosis eingestellt, kann die Dosis korrigiert werden, indem die Dosierhülse 30 wieder zurück in den Gehäuseeinsatz 20 eingedreht wird. Die Kupplungshülse 40 ist weiterhin mittels des Halteelements 50 rotationsfest relativ zum Gehäuseeinsatz 20 gehalten. Bei der Rückdrehung werden über die steilen Flanken der Sägezähne 36 in der Dosierhülse 30 die distalen Arme 72 und damit die ganze Klickscheibe 70 mitgedreht. Das hat zur Folge, dass die proximalen Arm 71 über die flache Flanke der Sägezähne 47 der Kupplungshülse 40 geführt wird, wodurch die proximalen Arme 71 ein Klickgeräusch und ein taktiles Signal erzeugt. Somit wird entweder durch die distalen Arme 72 (Erhöhung der Dosis) oder die proximalen Arme 71 (Reduzieren der Dosis) ein Klickgeräusch und ein taktiles Signal erzeugt.

Da die elastischen Arme der Klickscheibe 70 stets leicht in axialer Richtung komprimiert sind, wird eine Vorspannkraft erzeugt, mit der die Kupplungshülse 40 in der proximalen Dosierstellung gehalten wird bzw. in proximaler Richtung gegen einen durch den Ausschüttknopf 90 gebildeten Anschlag gedrückt wird. Somit ist die Kupplungshülse 40 durch die Vorspannkraft in der Dosierstellung gehalten, in welcher die Kupplungshülse 40 mit dem Halteelement 50 gekoppelt ist, indem die Zähne 41 am distalen Ende der Kupplungshülse 40 in die Nuten 54 im Halteelement 50 eingreifen. Da das Halteelement 50 stets rotationsfest am Gehäuseeinsatz 20 und Gehäuse 10 gehalten wird, ist auch die Kupplungshülse 40 in dieser Stellung drehfest relativ zum Gehäuseeinsatz 20 gehalten. Folglich kann sich beim Einstellen und Korrigieren einer Dosis, wenn die Dosierhülse 30 vorwärts oder rückwärts gedreht wird, die Kupplungshülse 40 nicht drehen.

Da das Halteelement 50 in axialer Richtung fest an der Dosierhülse 30 befestigt ist, wird das Halteelement 50 beim Einstellen einer Dosis zusammen mit der Dosierhülse 30 in axial in proximaler Richtung verschoben. Dadurch verbleibt die Kupplungshülse 40 in der gekoppelten Stellung mit dem Halteelement 50, auch wenn Kupplungshülse 40 und Halteelement 50 zusammen relativ zum Gehäuseeinsatz 20 und Gehäuse 10 axial verschoben werden. Mit dem Eingriff der Kupplungshülse 40 in das Halteelement 50 (siehe auch Figur 7) und die damit erfolgte rotative Kopplung der Kupplungshülse 40 zum Gehäuse 10 ist eine effiziente Rückdrehsicherung ermöglicht.

Dasselbe gilt beim Korrigieren einer eingestellten Dosis, also wenn die Dosierhülse 30 zurück in den Gehäuseeinsatz 20 geschraubt wird. In diesem Fall schiebt die Dosierhülse 30 über den axial zur Dosierhülse 30 fixierten Ausschüttknopf 90 die Kupplungshülse 40 axial in distaler Richtung wieder in den Gehäuseeinsatz 20 hinein. Da die Kupplungshülse 40 durch die Vorspannung relativ zum Halteelement 50 in Position gehalten wird und somit mit diesem in Eingriff verbleibt, kann sich die Kupplungshülse 40 auch beim Zurückdrehen der Dosierhülse 30 in distaler Richtung nicht relativ zum Gehäuseeinsatz 20 drehen.

Beim Einstellen und Korrigieren der Dosis, das bedeutet beim Herausdrehen der Dosierhülse 30 aus dem Gehäuseeinsatz 20 wie auch beim Einschrauben der Dosierhülse in den Gehäuseeinsatz 30 greifen die Verbindungsstege 44 der Kupplungshülse 40 nicht in die Nuten 32 im Innern der Dosierhülse 30 ein, wodurch Dosierhülse 30 und Kupplungshülse 40 relativ zueinander verdreht werden können, dargestellt in den Figuren 3 und 5.

Da die Kupplungshülse 40 beim Einstellen der Dosis und beim Korrigieren einer Dosis nicht rotieren kann, wird auch die Antriebshülse 60 nicht gedreht und die Kolbenstange 80 nicht angetrieben. Dadurch wird eine ungewollte Ausschüttung verhindert.

Die Stoppmutter 75 wird axial und rotationsfest in der Dosierhülse 30 geführt. Sie wird bei einer Drehung der Dosierhülse 30 zusammen mit der Dosierhülse 30 gedreht, wodurch die Stoppmutter 75 auf dem Aussengewinde 43 der Kupplungshülse 40 in proximaler Richtung geschraubt wird. Beim Korrigieren bzw. Zurückdrehen der Dosierhülse 30 wird die Stoppmutter 75 entsprechend wieder in distale Richtung geschraubt.

Um eine eingestellte Dosis auszuschütten, drückt der Benutzer in distaler Richtung auf den Ausschüttknopf 90. Der Ausschüttknopf 90 verschiebt sich durch die Druckkraft zusammen mit der Kupplungshülse 40 relativ zur Dosierhülse 30 in distaler Richtung. Die flexiblen Arme 71, 72 werden axial durch die stirnseitige Fläche des Flansches 45 der Kupplungshülse 40 und durch die stirnseitige Fläche der Dosierhülse 30 komprimiert. Da durch die distale Bewegung des Ausschüttknopfs 90 auch die Kupplungshülse 40 relativ zur Dosierhülse 30 nach distal verschoben wird, werden die Verbindungsstege 44 auf der Kupplungshülse 40 in die Nuten 32 in der Dosierhülse 30 eingeführt, wodurch die Kupplungshülse 40 rotativ mit der Dosierhülse 30 gekoppelt wird. Gleichzeitig werden bei dieser distalen Verschiebung der Kupplungshülse 40 die Zähne 41 am distalen Ende der Kupplungshülse 40 aus den Nuten 54 im Halteelement 50 herausgeschoben, so dass die Nuten 54 über dem zylinderförmigen Abschnitt 42 der Kopplungshülse 40 liegen und kein Eingriff mehr besteht, dargestellt in Figur 6. Dadurch ist die Kupplungshülse 40 nicht mehr rotativ mit dem Halteelement 50 und damit mit dem Gehäuseeinsatz 20 gekoppelt und die Kupplungshülse 40 kann sich relativ zum Gehäuseeinsatz 20 und relativ zum Gehäuse 10 drehen.

In einer anderen Ausführung wird die Kupplungshülse 40 zuerst vom Halteelement 50 rotativ freigegeben werden (durch Herausschieben der Zähne 41 aus den Nuten 54 des Halteelements 50) und erst anschliessend, wenn die Kupplungshülse 40 nicht mehr durch das Halteelement 50 gehalten ist, wird die Kupplungshülse 40 rotativ mit der Dosierhülse 30 gekoppelt. In einer weiteren Ausführung wird hingegen zuerst die Kupplungshülse 40 mit der Dosierhülse 30 rotativ gekoppelt und erst anschliessend an diese Kopplung wird die Kupplungshülse 40 vom Halteelement 50 rotativ freigegeben.

Wird die Kupplungshülse 40 weiter in distaler Richtung relativ zur Dosierhülse 30 verschoben, wird vom Flansch 45 der Kupplungshülse 40 in distaler Richtung die Druckkraft des Benutzers über die Klickscheibe 70 auf die stirnseitige Fläche der Dosierhülse 30 übertragen. Da die Dosierhülse 30 über ihr Aussengewinde im Gehäuseeinsatz 20 eingeschraubt ist, beginnt sich die Dosierhülse 30 unter der distal wirkenden Druckkraft mit einer Schraubbewegung in den Gehäuseeinsatz 20 einzuschrauben.

Da nun die Kupplungshülse 40 rotativ mit der Dosierhülse 30 gekoppelt ist und nicht länger über das Halteelement 50 rotationsfest gehalten ist, wird die Kupplungshülse 40 durch die drehende Dosierhülse 30 ebenfalls relativ zum Gehäuseeinsatz 20 und Gehäuse 10 gedreht. Dadurch wird auch die mit der Kupplungshülse 40 rotativ verbundene Antriebshülse 60 gedreht. Diese ist axial am Gehäuseeinsatz 20 gehalten und verschiebt sich somit bei der Drehung nicht axial zum Gehäuse 10. Die Rotation der Antriebshülse 60 treibt die mit ihr rotativ gekoppelte Kolbenstange 80 an, welche sich dadurch in das Innengewinde 27 des Gehäuseeinsatzes 20 in distaler Richtung einschraubt. Dadurch wird der Flansch 85 am distalen Ende der Kolbenstange 80 axial relativ zum Gehäuse 10 verschoben und kann somit den in der Karpule 12 befindliche Stopfen 13 in distaler Richtung relativ zur Karpule 12 verschieben. Infolge wird die medizinische Substanz aus der Karpule ausgeschüttet.

Die flexiblen Ratschenarme 63 der Antriebshülse 60 werden bei der Rotation der Antriebshülse 60 über die flachen Flanken der Sägezähne 25 des Gehäuseeinsatzes 20 bewegt, wodurch ein Klickgeräusch und ein taktiles Signal erzeugt wird. Bedingt durch die Sägezahnform können die Ratschenarme 63 nur in Ausschüttrichtung über die Sägezähne 25 geführt werden. In Gegenrichtung stehen die Ratschenarme 63 an den radialen Flanken der Sägezähne 25 an und verhindern somit eine Drehung der Antriebshülse 60. Dadurch kann die Kolbenstange 80 nur in Ausschüttrichtung bewegt werden. Die Stellung des Injektors 1, in der die eingestellte Dosis ausgeschüttet ist, ist in Figur 9 gezeigt.

Wie erwähnt, entsteht beim Ausschütten der Dosis durch die rotative Kopplung keine Relativbewegung zwischen Kupplungshülse 60 und Dosierhülse 30. Dadurch wird die Stoppmutter 75 mitgedreht, sie wird aber nicht relativ zur Kupplungshülse 40 verschoben. Das bedeutet, sie wird nicht auf dem Aussengewinde 43 in distaler oder proximaler Richtung bewegt. Somit wird die Stoppmutter 75 stets nur beim Einstellen oder Korrigieren relativ zur Kupplungshülse 40 und zur Dosierhülse 30 bewegt. Die Gewindesteigung und die Dimension der Stoppmutter 75 ist so ausgelegt, dass die Stoppmutter 75 am proximalen Ende des Aussengewindes der Kupplungshülse 40 mit einem Anschlag an einen radialen Anschlag an der Kupplungshülse 40 anstösst, wenn die maximale ausschüttbare Dosis eingestellt wurde. Dadurch wird sichergestellt, dass der Benutzer zwar mehrmals eine Dosis einstellen und ausschütten kann, dass jedoch nicht eine Dosis eingestellt werden kann, welche die Kapazität der Karpule 12 überschreitet.

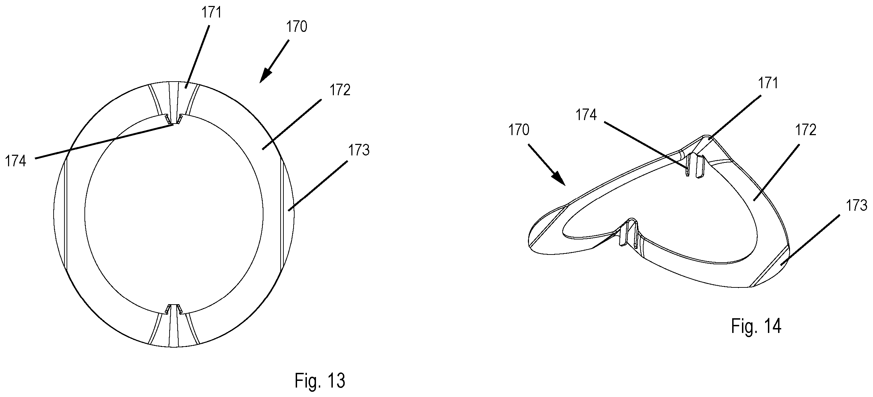

Der erfindungsgemässe Injektor 1 kann auch anders, als in der ersten Ausführung beschrieben, ausgeführt sein. In den Figuren 12 bis 14 ist der erfindungsgemässe Injektor in einer zweiten Ausführung dargestellt, in der der Injektor eine andere Klickscheibe 170 umfasst. Diese kann wiederum aus Metall gefertigt sein und im Zentrum ein Durchgang oder Öffnung aufweisen, so dass die Klickscheibe 170 einen Ring bildet. Allerdings stehen bei der Klickscheibe 170 gemäss den Figuren 12 bis 14 im Unterschied zur ersten Ausführung zwei Hälften 172 in einem Winkel von ungefähr 30° zu einer Einbauebene, welche senkrecht zur Längsachse des Gehäuses 10 steht. An ihrer äussersten Seite weisen die zwei Hälften 172 je einen abgewinkelten Abschnitt 173 auf, welcher parallel zur Einbauebene steht, erkennbar in Figur 14. Zudem laufen die beiden schrägen Hälften 172 zu einer Spitze 171 zusammen, welche eine Gegenform zu stirnseitigen Zähnen 131 der Dosierhülse 130 aufweist, siehe Figur 12. Die Klickscheibe 170 ist mittels zwei in axialer Richtung vom Ring abstehenden Laschen 174 Paaren gehalten, wobei die beiden Paare in Umfangrichtung um 180° versetzt und im Bereich der Spitze 171 des Rings angeordnet sind. Die Laschen 174 greifen in axiale Nuten in der Kupplungshülse 140 ein, wodurch die Klickscheibe 170 rotationsfest zur Kupplungshülse 140 gehalten ist.

Mit den abgewinkelten Abschnitten 173 stützt sich die Klickscheibe 170 an einem proximalen Flansch 141 der Kupplungshülse 140 ab (Figur 12). Die elastische Klickscheibe 170 ist im eingebauten Zustand leicht komprimiert und erzeugt dadurch eine Vorspannkraft, mit der sie die Kupplungshülse 140 in distaler Richtung in der Dosierstellung hält. Wie erwähnt, weist in der zweiten Ausführung die Dosierhülse 130 an ihrem proximalen Abschluss stirnseitig über den gesamten Umfang angeordnete Zähne 131 auf, wobei diese mit einem abgerundeten Übergang miteinander verbunden sind.

Beim Einstellen und Korrigieren einer Dosis wird die Spitze 171 der Klickscheibe 170 über die Zähne 131 bewegt und erzeugt dadurch ein Klickgeräusch und ein taktiles Signal. Da die Zähne 131 symmetrisch ausgebildet sind und mittels rundem Übergang miteinander verbunden sind, kann die Spitze 171 in beiden Drehrichtungen (Einstellen und Korrigieren) über die Zähne 131 bewegt werden. Die Klickscheibe 170 wird dabei in beiden Drehrichtungen mittels den Laschen 174 rotationsfest relativ zur Kupplungshülse 140 gehalten. Beim Ausschütten der Dosis, wenn die Kupplungshülse 130 relativ zur Dosierhülse 130 in distaler Richtung verschoben wird, wird die Klickscheibe 170 axial komprimiert, so dass die zwei angewinkelten Hälften 172 zur Einbauebene hin gedrückt werden. Das Koppeln der Kupplungshülse 140 mit der Dosierhülse 130 beim Ausschütten erfolgt wie oben in Zusammenhang mit der ersten Ausführung beschrieben.

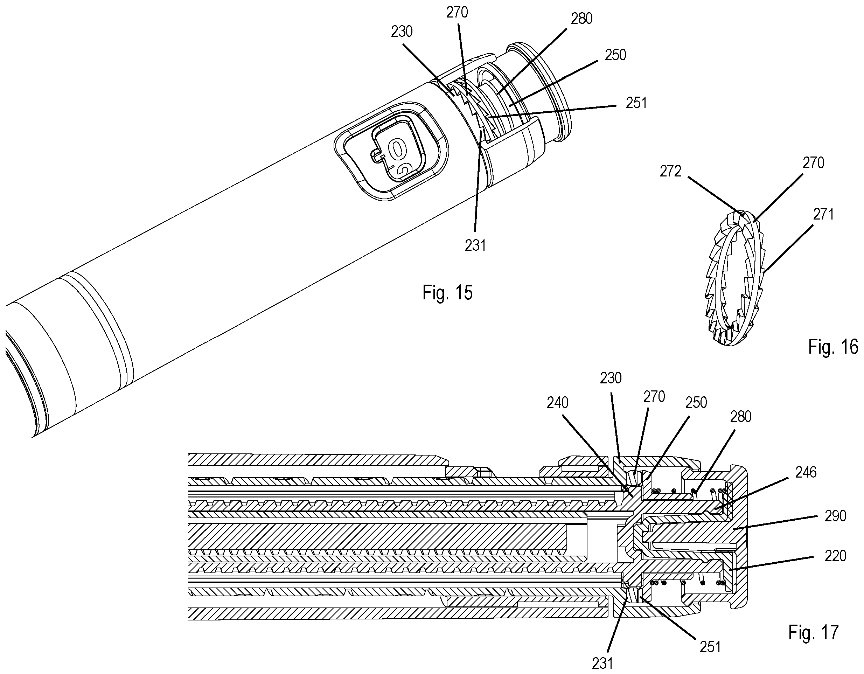

In den Figuren 15 bis 17 ist der proximale Bereich des erfindungsgemässen Injektors in einer dritten Ausführung dargestellt, welche eine Klickscheibe 270 umfasst. In Figur 15 ist zur besseren Ansicht ein Teil des Griffs der Dosierhülse nicht gezeichnet, so dass der Innenraum mit der Klickscheibe 270 ersichtlich ist. In dieser Ausführung weist der Injektor neben der Klickscheibe 270 zusätzlich eine Klickhülse 250 auf, welche auf einer distalen Seite einen Flansch aufweist, und auf einer proximalen Seite einen an den Flansch anschliessenden zylindrischen Abschnitt (Figur 17).

Die Dosierhülse 230 umfasst, wie in der ersten Ausführung beschrieben, auf einer stirnseitigen Fläche Sägezähne 231. Die Klickscheibe 270 kann, im Unterschied zur ersten Ausführung, aus Kunststoff gefertigt sein und weist sowohl auf der distalen wie auch auf der proximalen Seite Sägezähne 271, 272 auf, erkennbar in Figur 16. Dabei wirken die distalen Sägezähne 272 mit den Sägezähnen 231 der Dosierhülse 230 zusammen. Die Klickhülse 250 umfasst an der distalen stirnseitigen Fläche ihres Flansches Sägezähne 251, welche mit den proximalen Sägezähnen 271 der Klickscheibe 270 zusammenwirken können.

Wie in Figur 17 ersichtlich, weist bei dieser Ausführung der Injektor einen Knopfeinsatz 220 auf, welcher einen hohlzylindrischen Abschnitt und am proximalen Ende einen scheibenförmigen Abschnitt hat. Der hohlzylindrische Abschnitt befindet sich im proximalen Endelement 246 der Kupplungshülse 240 und der scheibenförmige Abschnitt schliesst an das proximale Ende der Kupplungshülse 240 an. Ein spitzförmiger distaler Abschluss des Knopfeinsatzes 220 stütz sich auf der Kupplungshülse 240 ab. Der hohlzylindrische Abschnitt des Knopfeinsatzes 220 umfasst eine umlaufende Ausformung, welche in einer Vertiefung im Endelement 246 der Kupplungshülse 240 aufgenommen ist. Der Knopfeinsatz 220 ist dadurch axialfest mit der Kupplungshülse 240 verschnappt. Der Auslöseknopf 290 weist, wie in der ersten Ausführung, eine distale Ausformung auf, welche sich am distalen Grund im hohlzylindrischen Abschnitt des Knopfeinsatzes 220 drehbar abstützt. Zudem ist der Auslöseknopf 290 wie bei der ersten Ausführung mit der Dosierhülse 230 verschnappt. Er ist relativ zum Knopfeinsatz 220 und relativ zur Dosierhülse 230 drehbar.

Zwischen dem Flansch der Klickhülse 250 und dem scheibenförmigen Abschnitt des Knopfeinsatzes 220 ist koaxial eine Klickfeder 280 eingebaut, ersichtlich in Figur 17. Die Klickfeder 280 stützt sich also mit ihrem proximalen Ende am Knopfeinsatz 220 und mit ihrem distalen Ende an der Klickscheibe 270 ab. Die Klickfeder 280 ist im eingebauten Zustand komprimiert, wodurch eine Vorspannkraft erzeugt wird, welche in distaler Richtung auf die Klickhülse 250 wirkt und deren Sägezähne 251 gegen die Sägezähne 271 der Klickscheibe 270 drückt. Die Klickscheibe 270 wiederum wird durch diese Vorspannkraft mit ihren distalen Sägezähnen 272 gegen die Sägezähne 231 der Dosierhülse 230 gedrückt.

Beim Einstellen einer Dosis wenn die Dosierhülse 230 aus dem Gehäuse gedreht wird, ergibt sich eine Relativbewegung der Dosierhülse 230 zur Klickscheibe 270 und die Sägezähne 231 der Dosierhülse 230 gleiten mit ihren flachen Flanken über die flachen Flanken der distalen Sägezähne 272 der Klickscheibe 270 und erzeugen dadurch ein Klickgeräusch und ein taktiles Signal. Die proximalen Sägezähne 271 der Klickscheibe 270 hingegen stossen mit den steilen Flanken an die steilen Flanken der Sägezähne 251 der Klickhülse 250, wodurch eine Relativbewegung zwischen Klickscheibe 270 und Klickhülse 250 verhindert wird.

Wird eine eingestellte Dosis korrigiert, indem die Dosierhülse 230 zurück in den Gehäuseeinsatz geschraubt wird, greifen die Sägezähne 231 der Dosierhülse 230 und diejenigen der Klickscheibe 270 so ineinander, dass eine Relativbewegung verhindert wird. Allerdings dreht sich in diesem Fall die Klickscheibe 270 zusammen mit der Dosierhülse 230 relativ zur Klickhülse 250, da die flachen Flanken der proximalen Sägezähne 271 der Klickscheibe 270 über die flachen Flanken der Klickhülse 250 gleiten und somit ebenfalls ein Klickgeräusch und ein taktiles Signal erzeugen. Die Klickfeder 280 stellt sicher, dass die Sägezähne 231 der Dosierhülse 230, der Klickscheibe 270 und der Klickhülse 250 stets aufeinander gedrückt werden und bei einer Relativbewegung der Sägezähne das Klickgeräusch erzeugt wird.

Beim Ausschütten einer Dosis wird, wie in der ersten Ausführung beschrieben, der Ausschüttknopf 290 zusammen mit der Kupplungshülse 240 relativ zur Dosierhülse 230 in distaler Richtung entgegen der Vorspannkraft der Klickfeder 280 verschoben. Da sich der Ausschüttknopf 290 axial am Knopfeinsatz 220 abstützt und dieser an der Kupplungshülse 240 anliegt, wird auch der Knopfeinsatz 220 verschoben. Bei der Ausschüttung rotiert die Dosierhülse 230, die mit der Dosierhülse 230 gekoppelte Kupplungshülse 240 wie auch die Klickscheibe 270, die Klickhülse 250, die Klickfeder 280 und der Knopfeinsatz 220 relativ zum Gehäuse und relativ zum Ausschüttknopf 290. Der Knopfeinsatz 220 verhindert somit, dass ein mit der Klickfeder 280 in Kontakt stehendes Element relativ zur Klickfeder 280 rotiert, was eine erhöhte Reibung zur Folge hätte.

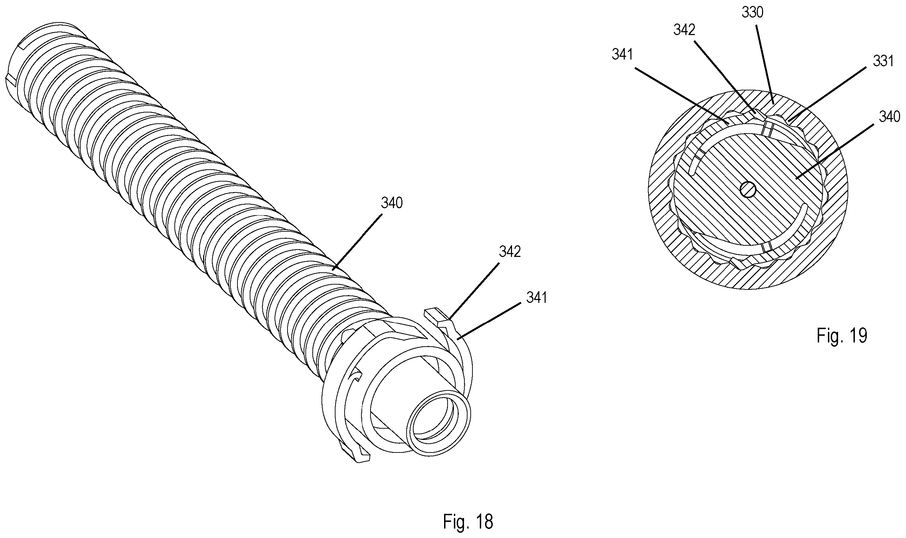

Die Figuren 18 und 19 zeigen eine vierte Ausführung, wobei Figur 18 eine perspektivische Ansicht der Kupplungshülse 340 und Figur 19 eine Schnittansicht in quer zur Längsachse durch die Kupplungshülse 340 und die Dosierhülse 330 im Bereich von radialen Armen 341 zeigt. In dieser vierten Ausführung umfasst der erfindungsgemässe Injektor keine Klickscheibe. Stattdessen sind direkt an der Kupplungshülse 340 die zwei radialen, elastischen Arme 341 ausgebildet. Die Arme 341 sind in einem proximalen Bereich an der Kupplungshülse 340 angebracht und umfassen am abstehenden Ende der Arme 341 eine radiale Ausbuchtung 342.

Wie in Figur 19 ersichtlich, weist die Dosierhülse 330 in dieser Ausführung auf ihrer Innenseite radiale Zähne 331 auf, mit denen die radialen Ausbuchtungen 342 der Arme 341 zusammenwirken können. Die Zähne 331 sind in Umfangrichtung nebeneinander angeordnet und mittels eines abgerundeten Übergangs miteinander verbunden. Dadurch können die Arme 341 mit den Ausbuchtungen 342 in beiden Drehrichtungen (Einstellen und Korrigieren einer Dosis) über die einzelnen Zähne 331 und Zwischenräume bewegt werden. Da die Arme 341 elastisch sind und mit einer Vorspannkraft gegen die Zähne 331 gedrückt werden, wird jedes Mal ein Klickgeräusch und ein taktiles Signal erzeugt.

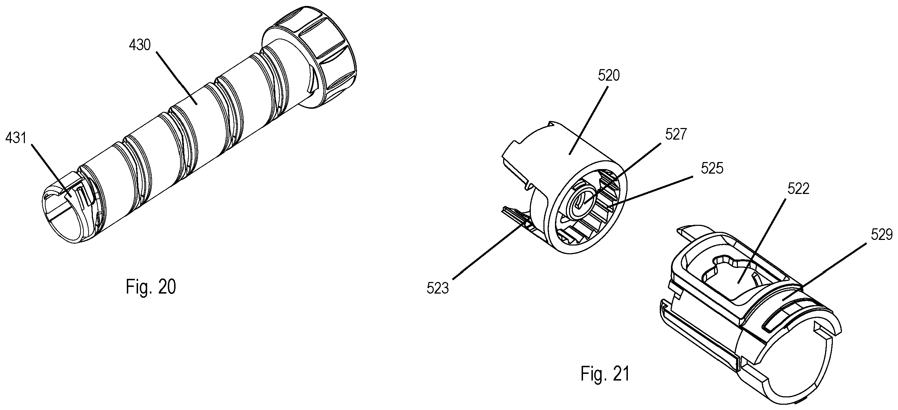

In der Figur 20 ist eine perspektivische Ansicht einer Variante der Dosierhülse 430 gezeigt. Im Unterschied zur ersten Ausführung ist bei dieser Variante anstelle eines radialen Stegs, welcher das Herausdrehen der Dosierhülse 430 aus dem Gehäuse begrenzt, ein elastisches Element in der Form einer flexiblen Lasche oder eines flexiblen Arms 431 vorgesehen. Ist die Dosierhülse 430 maximal aus dem Gehäuse herausgedreht, schlägt der abstehende Arm 431 an einen Anschlag im Gehäuseeinsatz an.

Zudem kann auch die Antriebshülse anders als in der ersten Ausführung beschrieben, ausgeführt sein. Beispielsweise kann für die axiale Halterung der Antriebshülse relativ zum Gehäuseeinsatz die Antriebshülse anstelle einer Schnappverbindung mit anderen Elementen axial fixiert werden. In einer Variante kann das Gehäuse eine radial zum Zentrum des Gehäuses weisende Wandung oder Rippe aufweisen, welche zumindest einen Teil des distalen Bereichs der Antriebshülse umschliesst, so dass die Antriebshülse an einer Verschiebung in proximale Richtung gehindert wird. In distaler Richtung kann ein Anschlag oder eine Wandung des Gehäuseeinsatzes eine Verschiebung der Antriebshülse verhindern. So ist die Antriebshülse axial unbeweglich, aber dennoch drehbar relativ zum Gehäuseeinsatz und zum Gehäuse gelagert.

Ferner kann der erfindungsgemässe Injektor einen Gehäuseeinsatz aufweisen, welcher anders ausgeführt ist als in der ersten Ausführung beschrieben. Figur 21 zeigt eine Variante, in welcher der Gehäuseeinsatz mehrteilig ausgebildet ist und einen distalen Gehäuseeinsatz 520 und einen proximalen Gehäuseeinsatz 529 umfasst. Der distale Gehäuseeinsatz 520 weist radiale zum Zentrum ragende Rippen 523 auf, zur Aufnahme der Karpule. Zudem umfasst der distale Gehäuseeinsatz 520 ein Innengewinde 527, in welches die Kolbenstange eingeschraubt werden kann. Der proximale Gehäuseeinsatz 529 umfasst ein Innengewinde, in das die Dosierhülse eingeschraubt werden kann und eine Öffnung in radialer Richtung, durch die die Skala der Dosierhülse von aussen ersichtlich ist. Weiter umfasst der proximale Gehäuseeinsatz im Innern einen radialen Steg, welcher als Anschlag dient, um ein Herausdrehen der Dosierhülse zu begrenzen. Der distale und der proximale Gehäuseeinsatz 520, 529 können jeweils mittels einer Schnappverbindung im Innenraum des Gehäuses verschnappt werden, so dass die Gehäuseeinsätze 520, 529 axial und rotativ am Gehäuse gehalten sind. Da bei dieser Ausführung der zweiteilige Gehäuseeinsatz keine Nut als Führung für das Halteelement bilden kann, ist die Nut direkt in der Innenwandung des Gehäuses vorgesehen.

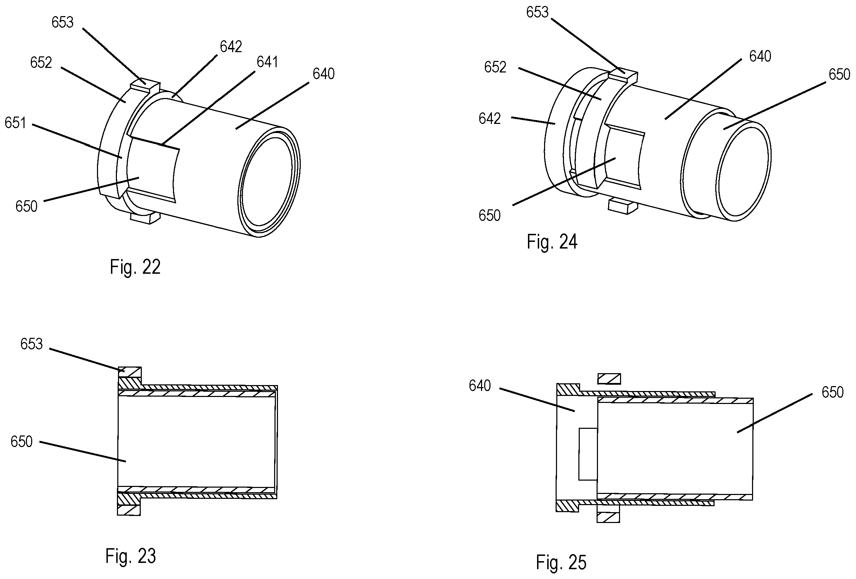

In einer weiteren Ausführung ist das Halteelement 650 und die Kupplungshülse 640 anders ausgebildet als oben beschrieben. Das Halteelement 650 umfasst in dieser Ausführung am distalen Ende zwei radiale Ratschenarme 652, welche je an ihrem Ende einen Nocken 653 aufweisen. Die Ratschenarme 652 sind dabei mittels einer radialen Wandung oder Steg 651 am zylinderförmigen Grundkörper des Halteelements 650 angebracht. Diese Ausführung ist in den Figuren 22 - 25 schematisch dargestellt. Dabei ist nur der distale Endbereich der Kupplungshülse 640 dargestellt. Diese weist im Unterschied zur oben beschriebenen Ausführung am distalen Ende einen umlaufenden Kragen 642 auf. Zudem umfasst die Kupplungshülse 640 in ihrem Zylinder zwei radiale Öffnungen 641, durch welche die radialen Stege 651 des Halteelements hindurchragen, so dass sich die Ratschenarme 652 ausserhalb einer Oberfläche der Kupplungshülse 640 befinden. Im Innern der Kupplungshülse 640 befindet sich die Antriebshülse, welche in den Figuren 22- 25 nicht dargestellt ist. Im Unterschied zur oben beschriebenen Ausführung weist die Antriebshülse keine Ratschenarme auf. Das Gehäuse (nicht gezeigt) weist auf seiner Innenseite in Längsrichtung mehrere über den Umfang verteilte axiale Nuten auf, in welche die Nocken 653 eingreifen können.

Beim Einstellen und Korrigieren einer Dosis liegen die Ratschenarme 652 auf dem Kragen 642 der Kupplungshülse 640 auf, ersichtlich in den Figuren 22 und 23. Die Ratschenarme 652 sind durch den Kragen blockiert und können sich nicht in radialer Richtung bewegen. Die Nocken 653 der Ratschenarme 652 werden in den Nuten des Gehäuses geführt. Dadurch kann sich die Kupplungshülse 640 beim Einstellen und Korrigieren der Dosis nicht relativ zum Gehäuse drehen, jedoch axial zusammen mit dem Halteelement 650 verschieben. Beim Ausschütten wird die Kupplungshülse 640, wie beim ersten Ausführungsbeispiel beschrieben, relativ zur Dosierhülse in distaler Richtung verschoben. Dadurch wird der Kragen 642 der Kupplungshülse 640 unter den Ratschenarme 652 distal weggeschoben, wodurch die Ratschenarme 652 radial frei sind und elastisch nach innen bewegt werden können, dargestellt in den Figuren 24 und 25. Die Kupplungshülse 640 ist dadurch rotativ zum Gehäuse freigegeben und rotiert beim Ausschütten relativ zum Gehäuse. Da die radialen Stege 651 und damit die Ratschenarme 652 mittels der Öffnungen 641 der Kupplungshülse 640 mitgenommen werden, bewegen sich die Ratschenarme 652 mit den Nocken 653 über die Nuten im Gehäuse und erzeugen dadurch ein taktiles und/oder akustisches Klicksignal während dem Ausschütten.

### BEZUGSZEICHENLISTE

| | | | | | |
|---|---|---|---|---|---|
| 1 | Injektor | 48 | Stege | 173 | Abschnitt |
| 2 | Gehäuse | | | 174 | Laschen |
| 11 | Schutzkappe | 50 | Halteelement | | |
| 12 | Karpule | 51 | distaler Abschnitt | 220 | Knopfeinsatz |
| 13 | Stopfen | 52 | proximaler Abschnitt | 230 | Dosierhülse |
| 15 | Karpulenhalter | 53 | Ausformung | 231 | Sägezähne |
| | | 54 | Nuten | 240 | Kupplungshülse |
| 20 | Gehäuseeinsatz | | | 246 | Endelement |
| 21 | Ausnehmung | 60 | Antriebshülse | 250 | Klickhülse |
| 22 | Öffnung | 61 | Stege | 251 | Sägezähne |
| 23 | Rippen | 62 | Nut | 270 | Klickscheibe |
| 24 | Wulst | 63 | Ratschenarme | 271 | Sägezähne |
| 25 | Sägezähne | | | 272 | Sägezähne |
| 26 | Nocken | 70 | Klickscheibe | 280 | Klickfeder |
| 27 | Innengewinde | 71 | proximale Arme | 290 | Ausschüttknopf |
| 28 | Innengewinde | 72 | distale Arme | 330 | Dosierhülse |
| | | 75 | Stoppmutter | 331 | Zähne |
| 30 | Dosierhülse | 76 | Stege | 340 | Kupplungshülse |
| 31 | Griff | | | 341 | Arm |
| 32 | Nuten | 80 | Kolbenstange | 342 | Ausbuchtung |
| 33 | Kragen | 81 | Absatz | 430 | Dosierhülse |
| 34 | Steg | 82 | Abschluss | 431 | Arm |
| 35 | Absatz | 85 | Flansch | 520 | distaler Einsatz |
| 36 | Sägezähne | | | 522 | Öffnung |
| 37 | Wulst | 90 | Auslöseknopf | 523 | Rippe |
| 38 | Stopphülse | 91 | Ausformung | 525 | Sägezähne |
| | | 92 | Kragen | 527 | Innengewinde |
| 40 | Kupplungshülse | | | 529 | prox. Einsatz |
| 41 | Zähne | 130 | Dosierhülse | 640 | Kupplungshülse |
| 42 | zylinderförmiger Abschnitt | 131 | Zähne | 641 | Öffnung |
| 43 | Aussengewinde | 140 | Kupplungshülse | 642 | Kragen |
| 44 | Verbindungsstege | 141 | Flansch | 650 | Halteelement |
| 45 | Flansch | 170 | Klickscheibe | 651 | radialer Steg |
| 46 | Endabschnitt | 171 | Spitze | 652 | Ratschenarm |
| 47 | Sägezähne | 172 | Hälfte | 653 | Nocken |

## Patentansprüche

1. Eine Dosiereinrichtung für eine Injektionsvorrichtung (1) zum Ausschütten einer Dosis eines Produkts umfassend
ein Gehäuse (10) mit einer Längsachse,
ein Dosiseinstellelement (30) zum Einstellen der Dosis,
ein Halteelement (50) und
eine Kupplungshülse (40) zum Antreiben einer Antriebseinrichtung zum Ausschütten der Dosis, wobei sich das Dosiseinstellelement (30), das Halteelement (50) und die Kupplungshülse (40) im Gehäuse (10) befinden und wobei
a) zum Einstellen und Korrigieren der Dosis das Dosiseinstellelement (30) und die Kupplungshülse (40) relativ zum Gehäuse (10) in Richtung Längsachse bewegbar sind und die Kupplungshülse (40) mittels Verzahnung zwischen Kupplungshülse (40) und Halteelement (50) rotationsfest relativ zum Gehäuse (10) gehalten werden kann;
**dadurch gekennzeichnet, dass**
b) zum Ausschütten der Dosis die Verzahnung zwischen Kupplungshülse (40) und Halteelement (50), durch Verschieben der Kupplungshülse (40) relativ zum Halteelement (50) in Richtung der Längsachse, aufgehoben wird, so dass die Kupplungshülse (40) relativ zum Gehäuse (10) rotieren kann, wobei die Dosiereinrichtung im Gehäuse (10) eine Führung umfasst, in welcher das Halteelement (50) beim Einstellen und Korrigieren der Dosis in Richtung der Längsachse relativ zum Gehäuse (10) verschiebbar ist und rotationsfest relativ zum Gehäuse (10) geführt ist.

2. Dosiereinrichtung nach Anspruch 1, **dadurch gekennzeichnet, dass** durch die Verschiebung der Kupplungshülse (40) zudem die Kupplungshülse (40) rotationsfest mit dem Dosiseinstellelement (30) koppelbar ist.

3. Dosiereinrichtung nach Anspruch 2, **dadurch gekennzeichnet, dass** die Kupplungshülse (40) mittels Verzahnung rotationsfest mit dem Dosiseinstellelement (30) koppelbar ist.

4. Dosiereinrichtung nach einem der Ansprüche 1 bis 3, **dadurch gekennzeichnet, dass** die Kupplungshülse (40) mittels Verzahnung rotationsfest relativ zum Halteelement (50) gehalten werden kann.

5. Dosiereinrichtung nach einem der Ansprüche 1 bis 4, **dadurch gekennzeichnet, dass** die Führung als Nut ausgebildet ist und das Halteelement (50) mittels einer Ausformung (53) rotationsfest relativ zum Gehäuse (10) in der Nut geführt ist.

6. Dosiereinrichtung nach einem der Ansprüche 1 bis 5, **dadurch gekennzeichnet, dass** das Halteelement (50) eine Öffnung aufweist, in welcher die Kupplungshülse (40) aufgenommen werden kann wobei das Halteelement (50) hülsenförmig oder ringförmig ausgebildet ist und mit seiner Aussenseite mit dem Gehäuse (10) oder einem Gehäuseeinsatz zusammen wirken kann und wobei die Innenseite der Öffnung mit einer Aussenseite der Kupplungshülse (40) zusammen wirken kann.

7. Dosiereinrichtung nach einem der Ansprüche 1 bis 6, **dadurch gekennzeichnet, dass** das Dosiseinstellelement (30) als Dosierhülse ausgebildet ist, welche axial am Halteelement (50) gehalten ist, so dass sich das Halteelement (50) zusammen mit der Dosierhülse in Richtung der Längsachse bewegen kann.

8. Dosiereinrichtung nach einem der Ansprüche 1 bis 7, **gekennzeichnet durch** ein elastisches Element (70), welches die Kupplungshülse (40) mit einer Vorspannkraft in einer Dosierstellung hält, in welcher die Kupplungshülse (40) rotationsfest relativ zum Halteelement (50) gehalten ist.

9. Dosiereinrichtung nach Anspruch 8, **dadurch gekennzeichnet, dass** das elastische Element (70) als Grundkörper mit mindestens zwei elastischen Armen (71, 72) ausgebildet ist, wobei der Grundkörper in einer Ebene senkrecht zur Längsachse ausgerichtet ist und dass ein erster der zwei Arme (71) in proximale Richtung und ein zweiter der zwei Arme (72) in distale Richtung weist.

10. Dosiereinrichtung nach Anspruch 9, **dadurch gekennzeichnet, dass** das Dosiseinstellelement (30) Zähne (36) umfasst und die Kupplungshülse (40) Zähne (47) umfasst, wobei zum Erzeugen eines akustischen oder taktilen Signals ein erster der mindestens zwei Arme (71, 72) mit den Zähnen (36) des Dosiseinstellelements (30) oder mit den Zähnen (47) der Kupplungshülse (40) zusammenwirken kann.

11. Dosiereinrichtung nach einem der Ansprüche 8 bis 10, **dadurch gekennzeichnet, dass** das Dosiseinstellelement (30) und die Kupplungshülse (40) in einem proximalen Bereich je einen Flansch aufweisen und das elastische Element (70) zwischen den Flanschen angeordnet ist.

12. Dosiereinrichtung nach einem der Ansprüche 1 bis 11, **gekennzeichnet durch** ein Gehäuseeinsatz (20), welcher eine Ausnehmung (21) umfasst, die die Führung für das Halteelement (50) bildet.

13. Dosiereinrichtung nach Anspruch 12 **dadurch gekennzeichnet, dass** der Gehäuseeinsatz (20) ein Innengewinde (27) aufweist, mit dem das Dosiseinstellelement (30) in Gewindeverbindung steht.

14. Injektionsvorrichtung zum Ausschütten einer Dosis umfassend einen Karpulenhalter zum Halten einer Karpule mit einer medizinischen Substanz, einer Nadel oder Kanüle und einer Dosiereinrichtung nach einem der Ansprüche 1 bis 13.

## Claims

1. Dosing apparatus for an injection device (1) for dispensing a dose of a product, which dosing apparatus comprises
a housing (10) having a longitudinal axis,
a dose setting element (30) for setting the dose,
a holding element (50), and
a coupling sleeve (40) for driving a drive device for dispensing the dose, wherein the dose setting element (30), the holding element (50) and the coupling sleeve (40) are located in the housing (10), and wherein
a) for setting and correcting the dose, the dose setting element (30) and the coupling sleeve (40) are movable relative to the housing (10) in the direction of the longitudinal axis, and the coupling sleeve (40) can be held in a rotationally fixed manner relative to the housing (10) by means of a toothed engagement between the coupling sleeve (40) and the holding element (50);
**characterized in that**
b) for dispensing the dose, the toothed engagement between the coupling sleeve (40) and the holding element (50) is disengaged by moving the coupling sleeve (40) relative to the holding element (50) in the direction of the longitudinal axis, so that the coupling sleeve (40) can rotate relative to the housing (10),
wherein
the dosing apparatus in the housing (10) comprises a guide means in which the holding element (50) can be moved in the direction of the longitudinal axis relative to the housing (10) when setting and correcting the dose, and in which the holding element is guided in a rotationally fixed manner relative to the housing (10).

2. Dosing apparatus according to claim 1, **characterized in that** by moving the coupling sleeve (40), the coupling sleeve (40) can also be coupled in a rotationally fixed manner to the dose setting element (30).

3. Dosing apparatus according to claim 2, **characterized in that** the coupling sleeve (40) can be coupled to the dose setting element (30) in a rotationally fixed manner by means of the toothed engagement.

4. Dosing apparatus according to any of claims 1 to 3,
**characterized in that** the coupling sleeve (40) can be held in a rotationally fixed manner relative to the holding element (50) by means of the toothed engagement.

5. Dosing apparatus according to any of claims 1 to 4,
**characterized in that** the guide means is designed as a groove and the holding element (50) is guided in the groove in a rotationally fixed manner relative to the housing (10) by means of a shaped portion (53).

6. Dosing apparatus according to any of claims 1 to 5,
**characterized in that** the holding element (50) has an opening in which the coupling sleeve (40) can be received, wherein the holding element (50) is sleeve-shaped or annular and the outer side of said holding element can interact with the housing (10) or with a housing insert, and wherein the inner side of the opening can interact with an outer side of the coupling sleeve (40).

7. Dosing apparatus according to any of claims 1 to 6,
**characterized in that** the dose setting element (30) is designed as a dosing sleeve which is held axially on the holding element (50), so that the holding element (50) can move together with the dosing sleeve in the direction of the longitudinal axis.

8. Dosing apparatus according to any of claims 1 to 7, **characterized by** an elastic element (70) which holds the coupling sleeve (40) with a preload force in a dosing position in which the coupling sleeve (40) is held in a rotationally fixed manner relative to the holding element (50).

9. Dosing apparatus according to claim 8, **characterized in that** the elastic element (70) is designed as a main body having at least two elastic arms (71, 72), wherein the main body is aligned in a plane perpendicular to the longitudinal axis, and **in that** a first of the two arms (71) points in the proximal direction and a second of the two arms (72) points in the distal direction.

10. Dosing apparatus according to claim 9, **characterized in that** the dose setting element (30) comprises teeth (36) and the coupling sleeve (40) comprises teeth (47), wherein a first of the at least two arms (71, 72) can interact with the teeth (36) of the dose setting element (30) or with the teeth (47) of the coupling sleeve (40) to generate an acoustic or tactile signal.

11. Dosing apparatus according to any of claims 8 to 10, **characterized in that** the dose setting element (30) and the coupling sleeve (40) each have a flange in a proximal region and the elastic element (70) is arranged between the flanges.

12. Dosing apparatus according to any of claims 1 to 11, **characterized by** a housing insert (20) which comprises a recess (21) which forms the guide means for the holding element (50).

13. Dosing apparatus according to claim 12 **characterized in that** the housing insert (20) has an internal thread (27) with which the dose setting element (30) is in threaded connection.

14. Injection device for dispensing a dose, comprising a carpule holder for holding a carpule with a medical substance, a needle or cannula and a dosing apparatus according to any of claims 1 to 13.

## Revendications

1. Appareil de dosage pour un dispositif d'injection (1) pour la distribution d'une dose d'un produit comprenant
un boîtier (10) comportant un axe longitudinal,
un élément de réglage de dose (30) permettant de régler la dose,
un élément de maintien (50) et
un manchon d'accouplement (40) pour l'entraînement d'un appareil d'entraînement pour la distribution de la dose, dans lequel l'élément de réglage de dose (30), l'élément de maintien (50) et le manchon d'accouplement (40) se trouvent dans le boîtier (10) et dans lequel
a) pour le réglage et la correction de la dose, l'élément de réglage de dose (30) et le manchon d'accouplement (40) sont mobiles par rapport au boîtier (10) dans la direction de l'axe longitudinal et le manchon d'accouplement (40) peut être maintenu de manière fixe en rotation par rapport au boîtier (10) au moyen d'une denture entre le manchon d'accouplement (40) et l'élément de maintien (50) ;
**caractérisé en ce que**
b) pour la distribution de la dose, la denture entre le manchon d'accouplement (40) et l'élément de maintien (50) est supprimée en faisant coulisser le manchon d'accouplement (40) par rapport à l'élément de maintien (50) dans la direction de l'axe longitudinal, de sorte que le manchon d'accouplement (40) peut tourner par rapport au boîtier (10),
dans lequel
l'appareil de dosage comprend dans le boîtier (10) un guidage dans lequel l'élément de maintien (50) peut coulisser par rapport au boîtier (10) dans la direction de l'axe longitudinal lors du réglage et de la correction de la dose et est guidé de manière fixe en rotation par rapport au boîtier (10).

2. Appareil de dosage selon la revendication 1, **caractérisé en ce qu'au** moyen du coulissement du manchon d'accouplement (40), le manchon d'accouplement (40) peut en outre être accouplé de manière fixe en rotation à l'élément de réglage de dose (30).

3. Appareil de dosage selon la revendication 2, **caractérisé en ce que** le manchon d'accouplement (40) peut être accouplé de manière fixe en rotation avec l'élément de réglage de dose (30) au moyen d'une denture.

4. Appareil de dosage selon l'une des revendications 1 à 3,
**caractérisé en ce que** le manchon d'accouplement (40) peut être maintenu de manière fixe en rotation par rapport à l'élément de maintien (50) au moyen d'une denture.

5. Appareil de dosage selon l'une des revendications 1 à 4,
**caractérisé en ce que** le guidage est réalisé sous la forme d'une rainure et l'élément de maintien (50) est guidé dans la rainure de manière fixe en rotation par rapport au boîtier (10) au moyen d'un bossage (53).

6. Appareil de dosage selon l'une des revendications 1 à 5,
**caractérisé en ce que** l'élément de maintien (50) présente une ouverture dans laquelle le manchon d'accouplement (40) peut être reçu, dans lequel l'élément de maintien (50) est réalisé en forme de manchon ou en forme d'anneau et peut coopérer au moyen de son côté extérieur avec le boîtier (10) ou un insert de boîtier et dans lequel le côté intérieur de l'ouverture peut coopérer avec un côté extérieur du manchon d'accouplement (40).

7. Appareil de dosage selon l'une des revendications 1 à 6,
**caractérisé en ce que** l'élément de réglage de dose (30) est réalisé sous la forme d'un manchon de dosage qui est maintenu axialement sur l'élément de maintien (50), de sorte que l'élément de maintien (50) peut se déplacer conjointement avec le manchon de dosage dans la direction de l'axe longitudinal.

8. Appareil de dosage selon l'une des revendications 1 à 7, **caractérisé par** un élément élastique (70) qui maintient le manchon d'accouplement (40) avec une force de précontrainte dans une position de dosage dans laquelle le manchon d'accouplement (40) est maintenu de manière fixe en rotation par rapport à l'élément de maintien (50).

9. Appareil de dosage selon la revendication 8, **caractérisé en ce que** l'élément élastique (70) est réalisé sous forme de corps de base comportant au moins deux bras élastiques (71, 72), dans lequel le corps de base est orienté dans un plan perpendiculaire à l'axe longitudinal et **en ce qu'**un premier bras parmi les deux bras (71) est orienté dans la direction proximale et un second bras parmi les deux bras (72) est orienté dans la direction distale.

10. Appareil de dosage selon la revendication 9, **caractérisé en ce que** l'élément de réglage de dose (30) comprend des dents (36) et le manchon d'accouplement (40) comprend des dents (47), dans lequel, pour générer un signal acoustique ou tactile, un premier bras parmi les au moins deux bras (71, 72) peut coopérer avec les dents (36) de l'élément de réglage de dose (30) ou avec les dents (47) du manchon d'accouplement (40).

11. Appareil de dosage selon l'une des revendications 8 à 10,
**caractérisé en ce que** l'élément de réglage de dose (30) et le manchon d'accouplement (40) présentent respectivement une bride dans une zone proximale et l'élément élastique (70) est disposé entre les brides.

12. Appareil de dosage selon l'une des revendications 1 à 11, **caractérisé par** un insert de boîtier (20) qui comprend un évidement (21) qui forme le guidage pour l'élément de maintien (50).

13. Appareil de dosage selon la revendication 12 **caractérisé en ce que** l'insert de boîtier (20) présente un filetage intérieur (27) au moyen duquel l'élément de réglage de dose (30) est en liaison par filetage.

14. Dispositif d'injection pour la distribution d'une dose comprenant un support pour carpule permettant de maintenir une carpule comportant une substance médicale, une aiguille ou une canule et un appareil de dosage selon l'une des revendications 1 à 13.
